(19) **Europäisches Patentamt / European Patent Office / Office européen des brevets**

(11) **EP 4 530 449 B1**

(12) **EUROPEAN PATENT SPECIFICATION**

(45) Date of publication and mention
of the grant of the patent:
**05.08.2026 Bulletin 2026/32**

(21) Application number: **24202634.2**

(22) Date of filing: **25.09.2024**

(51) International Patent Classification (IPC):
*F01M 11/10* (2006.01)    *G01N 33/28* (2006.01)

(52) Cooperative Patent Classification (CPC):
**F01M 11/10;** F01M 2011/14; F01M 2011/1426;
F16N 2260/18; G01N 33/2888

(54) **SYSTEMS AND METHODS FOR ESTIMATING A REMAINING USEFUL LIFE OF VEHICLE ENGINE OIL**

SYSTEME UND VERFAHREN ZUR SCHÄTZUNG DER VERBLEIBENDEN LEBENSDAUER VON FAHRZEUGMOTORÖL

SYSTÈMES ET PROCÉDÉS D'ESTIMATION D'UNE DURÉE DE VIE UTILE RESTANTE D'HUILE DE MOTEUR DE VÉHICULE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.09.2023 US 202363540413 P**

(43) Date of publication of application:
**02.04.2025 Bulletin 2025/14**

(73) Proprietor: **GEOTAB Inc.**
**Oakville, ON L6H 7V2 (CA)**

(72) Inventors:
• **Raj, Lourd Arun**
 **St. Laurent, H4L 1X9 (CA)**
• **Arezza, Giordano Aldo**
 **Maple, L6A 2P6 (CA)**
• **Lewis, Daniel J.**
 **Cambridge, N3C 1H8 (CA)**
• **Santorelli, Michael Angelo David**
 **Concord, L4K 3G7 (CA)**
• **Spencer, James Allan**
 **Toronto, M5V 2W8 (CA)**

(74) Representative: **von Zmuda-Trzebiatowski, Margarethe**
**Geotab GmbH**
**Kaiserstraße 100**
**52134 Herzogenrath (DE)**

(56) References cited:
CN-A- 112 440 903    CN-B- 111 091 244
US-A- 5 604 441    US-A1- 2022 412 912

## Description

### TECHNICAL FIELD

The present disclosure generally relates to predictive vehicle maintenance. More specifically, the present disclosure relates to estimating the remaining useful life of vehicle engine oil.

### BACKGROUND

Today, many vehicles rely on computer-based systems (e.g., one or more processors) for their operation. As will be appreciated, such systems manage and/or produce many types of data associated with various aspects of the vehicle during the operation thereof that may generally be referred to as "telematics data". In more detail, telematics data may include any information, parameters, attributes, characteristics, and/or features associated with the vehicle, such as, but not limited to, location data, speed data, acceleration data, fluid level data, energy data, engine data, brake data, transmission data, odometer data, vehicle identifying data, error/diagnostic data, tire pressure data, seatbelt data, and airbag data.

For example, the computer-based systems may estimate a vehicle's engine oil quality so that an operator of the vehicle may be alerted when the engine oil should be changed. The vehicle engine oil quality may be estimated by the computer-based systems using, for example, a proprietary algorithm of the vehicle original equipment manufacturer (OEM). However, such proprietary algorithms are often configured to vehicle engine oil quality based on vehicle operational factors selected by the OEM. For example, the proprietary algorithms may be configured to estimate vehicle engine oil quality based on expected vehicle operational factors such as an expected engine oil type, expected operator driving habits, expected vehicle driving conditions, etc. However, if any of such vehicle operational factors change from those expected by the OEM, the proprietary algorithms may no longer accurately estimate the vehicle engine oil quality.

If the estimated vehicle engine oil quality is not accurate, an operator may be directed (e.g., by an indicator in the vehicle) to change the engine oil earlier, or later, than necessary. As will be appreciated, changing vehicle engine oil too early may lead to wasted engine oil, increased vehicle maintenance, which, in turn, may lead to increased vehicle downtime, etc. Changing vehicle engine oil too late, alternatively, may lead to damage to the vehicle engine and, as a result, unnecessary, and likely costly, vehicle maintenance. Changing vehicle engine oil too early or too late may therefore lead to increased vehicle operational costs, especially if a user is managing a vehicle fleet. Patent publication US 2022/412912 A1 discusses information that is useful for understanding the background of the invention.

A need therefore exists for improved systems and methods for predicting a remaining useful life of vehicle engine oil.

### SUMMARY

In one aspect, the present disclosure relates to a system for estimating a remaining useful life of vehicle engine oil, the system comprising: at least one data storage operable to store at telematics data originating from a plurality of vehicles, the telematics data comprising odometer data and engine oil quality data associated with each of the plurality of vehicles; and at least one processor in communication with the at least one data storage, the at least one processor operable to: receive the telematics data; determine, for one or more of the plurality of vehicles, a start point of a current oil cycle based on when an oil cycle event previously occurred by identifying within the engine oil quality data: a first series of engine oil quality datapoints, and a second series of engine oil quality datapoints immediately following the first series, a difference between a final datapoint of the first series and an initial datapoint of the second series being greater than a predetermined threshold; synchronize the odometer data and the engine oil quality data by associating odometer datapoints and engine oil quality datapoints reported within a same time period; determine a remaining useful vehicle engine oil distance by applying to synchronized odometer and engine oil quality data reported during the current oil cycle a regression model; and determine a remaining useful vehicle engine oil time by converting the remaining useful vehicle engine oil distance thereto based on an average difference in odometer datapoints reported during a selected time period, whereby the remaining useful life of vehicle engine oil is estimated based on vehicle operational factors associated with the current oil cycle, and independently from those of a previous oil cycle.

According to an embodiment, the initial datapoint of the second series is greater than the final datapoint of the first series.

According to an embodiment, the initial datapoint of the second series is less than the final datapoint of the first series.

According to an embodiment, an initial datapoint of the first series is greater than the final datapoint thereof, and the initial datapoint of the second series is greater than a final datapoint thereof.

According to an embodiment, each datapoint of the first series and the second series decreases relative to an immediately preceding datapoint.

According to an embodiment, each datapoint of the first series and the second series is greater than an immediately preceding datapoint within a predetermined threshold, less than the immediately preceding datapoint within a predetermined threshold, or equal to the immediately preceding datapoint.

According to an embodiment, the at least one processor is operable to determine the start point of the current oil cycle by: selecting engine oil quality data reported within a selected reporting period; and identifying within the engine oil quality data reported within the selected reporting period the first series and the second series.

According to an embodiment, the selected reporting period comprises at least 10 reported engine oil quality datapoints.

According to an embodiment, the at least one processor is operable to synchronize the odometer data and the engine oil quality data by associating the odometer datapoints with the engine oil quality datapoints reported within an hour of each other.

According to an embodiment, the at least one processor is operable to determine the remaining useful vehicle engine oil distance by: determining from the regression model at least a rate of degradation of the engine oil quality datapoints; determining, based on the rate of degradation, an odometer reading at which the engine oil quality datapoints indicate engine oil should be changed; and determining the remaining useful vehicle engine oil distance based on a difference between a most recent odometer datapoint and the odometer reading at which the engine oil quality datapoints indicate engine oil should be changed.

According to an embodiment, the at least one processor is further operable to determine confidence bounds for the rate of degradation based on a residual standard deviation thereof and a confidence coefficient.

According to an embodiment, the at least one processor is operable to: determine the confidence coefficient based on one or more previous oil cycles of the one or more of the plurality of vehicles, select the confidence coefficient based on one or more vehicle parameters, or a combination thereof.

According to an embodiment, the remaining useful vehicle engine oil distance and the remaining useful vehicle engine oil time each comprise a range based on the confidence bounds of the rate of degradation.

According to an embodiment, the telematics data further comprises vehicle identifying information associated with the plurality of vehicles; and the at least one processor is further operable to determine the remaining useful vehicle engine oil distance by: identifying, using the vehicle identifying information, a vehicle type of each of the one or more of the plurality of vehicles; selecting, for each of the one or more of the plurality of vehicles, a type of regression model based on the vehicle type of each thereof; and applying the regression model to the synchronized odometer and engine oil quality data reported during the current oil cycle.

According to an embodiment, the vehicle type comprises a passenger vehicle or an industrial vehicle.

According to an embodiment, the regression model comprises a linear regression model, a polynomial regression model, a linear degradation model, an exponential degradation model, or a combination thereof.

According to an embodiment, the at least one processor is operable to determine the remaining useful vehicle engine oil time based on an average daily difference in odometer datapoints over the selected time period.

According to an embodiment, the selected time period is an amount of time elapsed from the start point of the current oil cycle.

According to an embodiment, the at least one processor is operable to repeat the estimation of the remaining useful life of the vehicle engine oil at a predetermined cadence.

According to an embodiment, the at least one processor is further operable to send each remaining useful vehicle engine oil distance and each remaining useful vehicle engine oil time to the at least one data storage for storage thereon.

According to an embodiment, the at least one processor is operable to, if the first series and the second series are not present within the engine oil quality data, determine the start point of the current oil cycle to be a first reported engine oil quality datapoint.

In another aspect, the present disclosure relates to a method for estimating a remaining useful life of vehicle engine oil, the method comprising operating at least one processor to: receive telematics data originating from a plurality of vehicles, the telematics data comprising odometer data and engine oil quality data associated with each of the plurality of vehicles; determine, for one or more of the plurality of vehicles, a start point of a current oil cycle based on when an oil cycle event previously occurred by identifying within the engine oil quality data: a first series of engine oil quality datapoints, and a second series of engine oil quality datapoints immediately following the first series, a difference between a final datapoint of the first series and an initial datapoint of the second series being greater than a predetermined threshold; synchronize the odometer data and the engine oil quality data by associating odometer datapoints and engine oil quality datapoints reported within a same time period; determine a remaining useful vehicle engine oil distance by applying to synchronized odometer and engine oil quality data reported during the current oil cycle a regression model; and determine a remaining useful vehicle engine oil time by converting the remaining useful vehicle engine oil distance thereto based on an average difference in odometer datapoints reported during a selected time period, whereby the remaining useful life of vehicle engine oil is estimated based on vehicle operational factors associated with the current oil cycle, and independently from those of a previous oil cycle.

According to an embodiment, the initial datapoint of the second series is greater than the final datapoint of the first series.

According to an embodiment, the initial datapoint of the second series is less than the final datapoint of the first series.

According to an embodiment, an initial datapoint of the first series is greater than the final datapoint thereof, and the initial datapoint of the second series is greater than a final datapoint thereof.

According to an embodiment, each datapoint of the first series and the second series decreases relative to an immediately preceding datapoint.

According to an embodiment, each datapoint of the first series and the second series is greater than an immediately preceding datapoint within a predetermined threshold, less than the immediately preceding datapoint within a predetermined threshold, or equal to the immediately preceding datapoint.

According to an embodiment, the determining of the start point of the current oil cycle comprises operating the at least one processor to: select engine oil quality data reported within a selected reporting period; and identify within the engine oil quality data reported within the selected reporting period the first series and the second series.

According to an embodiment, the selected reporting period comprises at least 10 reported engine oil quality datapoints.

According to an embodiment, the synchronizing of the odometer data and the engine oil quality data comprises operating the at least one processor to associate the odometer datapoints with the engine oil quality datapoints reported within an hour of each other.

According to an embodiment, the determining of the remaining useful vehicle engine oil distance comprises operating the at least one processor to: determine from the regression model at least a rate of degradation of the engine oil quality datapoints; determine, based on the rate of degradation, an odometer reading at which the engine oil quality datapoints indicate engine oil should be changed; and determine the remaining useful vehicle engine oil distance based on a difference between a most recent odometer datapoint and the odometer reading at which the engine oil quality datapoints indicate engine oil should be changed.

According to an embodiment, the method further comprises operating the at least one processor to determine confidence bounds for the rate of degradation based on a residual standard deviation thereof and a confidence coefficient.

According to an embodiment, the method further comprises operating the at least one processor to: determine the confidence coefficient based on one or more previous oil cycles of the one or more of the plurality of vehicles, select the confidence coefficient based on one or more vehicle parameters, or a combination thereof.

According to an embodiment, the remaining useful vehicle engine oil distance and the remaining useful vehicle engine oil time each comprise a range based on the confidence bounds of the rate of degradation.

According to an embodiment, the telematics data further comprises vehicle identifying information associated with the plurality of vehicles; and the determining of the remaining useful vehicle engine oil distance further comprises operating the at least one processor to: identifying, using the vehicle identifying information, a vehicle type of each of the one or more of the plurality of vehicles; selecting, for each of the one or more of the plurality of vehicles, a type of regression model based on the vehicle type of each thereof; and applying the regression model to the synchronized odometer and engine oil quality data reported during the current oil cycle.

According to an embodiment, the vehicle type comprises a passenger vehicle or an industrial vehicle.

According to an embodiment, the regression model comprises a linear regression model, a polynomial regression model, a linear degradation model, an exponential degradation model, or a combination thereof.

According to an embodiment, the average difference in odometer datapoints over the selected time period is an average daily difference in odometer datapoints.

According to an embodiment, the selected time period is an amount of time elapsed from the start point of the current oil cycle.

According to an embodiment, the method comprises operating the at least one processor to repeat the estimation of the remaining useful life of the vehicle engine oil at a predetermined cadence.

According to an embodiment, the method further comprises operating the at least one processor to send each remaining useful vehicle engine oil distance and each remaining useful vehicle engine oil time to at least one data storage for storage thereon.

According to an embodiment, the method comprises operating the at least one processor to, if the first series and the second series are not present within the engine oil quality data, determine the start point of the current oil cycle to be a first reported engine oil quality datapoint.

In another aspect, the present disclosure relates to a non-transitory computer readable medium having instructions stored thereon executable by at least one processor to implement the methods described herein.

Other aspects and features of the systems and methods of the present disclosure will become apparent to those ordinarily skilled in the art upon review of the following description of specific embodiments.

**BRIEF DESCRIPTION OF THE DRAWINGS**

These and other features of the present disclosure will become more apparent in the following detailed description in which reference is made to the appended drawings. The appended drawings illustrate one or more embodiments of the present disclosure by way of example only and are not to be construed as limiting the scope of the present disclosure.

FIG. 1 is a block diagram of various components interacting with an example fleet management system according to an embodiment of the present disclosure.

FIG. 2 is a block diagram of an example fleet management system interacting with an example telematics device and an example vehicle, according to an embodiment of the present disclosure.

FIG. 3 is a block diagram of an example computing device interacting with an example fleet management system, according to an embodiment of the present disclosure.

FIG. 4 is a flowchart of an example method for estimating the remaining useful life of vehicle engine oil, according to an embodiment of the present disclosure.

FIG. 5 show different examples of engine oil quality data received from vehicles, according to an embodiment of the present disclosure.

FIG. 6 shows an example of determining remaining useful engine oil distance from synchronized odometer and engine oil quality data, according to an embodiment of the present disclosure.

## DETAILED DESCRIPTION

Combustion engine vehicles require engine oil for their operation. Engine oil is generally used to lubricate moving components of the engine to reduce friction therebetween and maintain engine performance. However, over time, engine oil will deteriorate (e.g., due to accumulation of dust and dirt, oxidization of the oil, etc.), reducing its effectiveness as a lubricant, which may lead to engine damage. The engine oil must therefore be replaced (or "changed") with clean engine oil before it deteriorates to a point where the engine may be damaged.

Many vehicles today estimate their engine oil quality - i.e., the remaining useful life of the engine oil, or, put differently, when the engine oil should be changed. Typically, computer-based systems of the vehicles employ a proprietary algorithm to estimate the engine oil quality based on vehicle operational factors expected by the original equipment manufacturer (OEM). Examples of such expected vehicle operational factors include, but are not limited to, an expected engine oil type (e.g., synthetic engine oil, semi-synthetic engine oil, or conventional engine oil), expected operator driving habits, expected vehicle driving conditions, etc. However, if any of the vehicle operational factors change from those expected by the OEM (e.g., an operator uses a different type of engine oil), the proprietary algorithm may no longer accurately estimate the vehicle engine oil quality.

If vehicle engine oil is not accurately estimated, an operator of the vehicle (or a fleet manager managing a vehicle fleet) may be directed to (e.g., by an indicator in the vehicle, a fleet management system, etc.) change the engine oil too early (e.g., before the engine oil has deteriorated) or too late (e.g., after the engine oil has deteriorated to the point where the vehicle engine may be damaged). As a result, non-deteriorated vehicle engine oil may be wasted, vehicle maintenance may be performed more often than required, the vehicle engine may be damaged, vehicle downtime may be increased, etc. Thus, changing engine oil at an incorrect time may lead to a significant increase in costs for a vehicle operator. As will be appreciated, such costs may increase by a significant magnitude for users managing vehicle fleets.

It is therefore an object of the present disclosure to provide improved systems and methods for estimating the remaining useful life of vehicle engine oil.

For example, in some embodiments, the systems and methods of the present disclosure may avoid the shortcomings of the conventional techniques (e.g., proprietary algorithms employed by vehicle OEMs). In more detail, the systems and methods described herein may advantageously account for any changes to vehicle operational factors when estimating the remaining useful life of vehicle engine oil. That is, unlike the conventional techniques that estimate the remaining useful life of engine oil based on vehicle operational factors expected by the OEM, the systems and methods of the present disclosure may use actual, current vehicle operational factors in their estimation. As a result, the estimation of the remaining useful life of vehicle engine oil determined by the systems and methods of the present disclosure may be more accurate than the conventional techniques.

By having a more accurate estimation of the remaining useful life of the vehicle engine oil, an operator (or, for example, a vehicle fleet manager) may avoid the difficulties associated with changing engine oil too early and/or too late described herein. For example, an operator may avoid wasting engine oil still having useful life remaining, may avoid engine damage caused by continuing to use engine oil having no useful life remaining, as well as vehicle downtime and costs associated with each thereof. As will be appreciated, avoiding such difficulties may be particularly useful for a user managing a vehicle fleet, as any vehicle downtime and/or maintenance costs incurred from an inaccurate remaining useful engine oil life estimation may be significant when multiplied across, for example, hundreds or even thousands of vehicles.

Additional advantages will be discussed below and will be readily apparent to those of ordinary skill in the art upon reading the present disclosure.

Reference will now be made in detail to example embodiments of the disclosure, wherein numerals refer to like components, examples of which are illustrated in the accompanying drawings that further show example embodiments, without limitation.

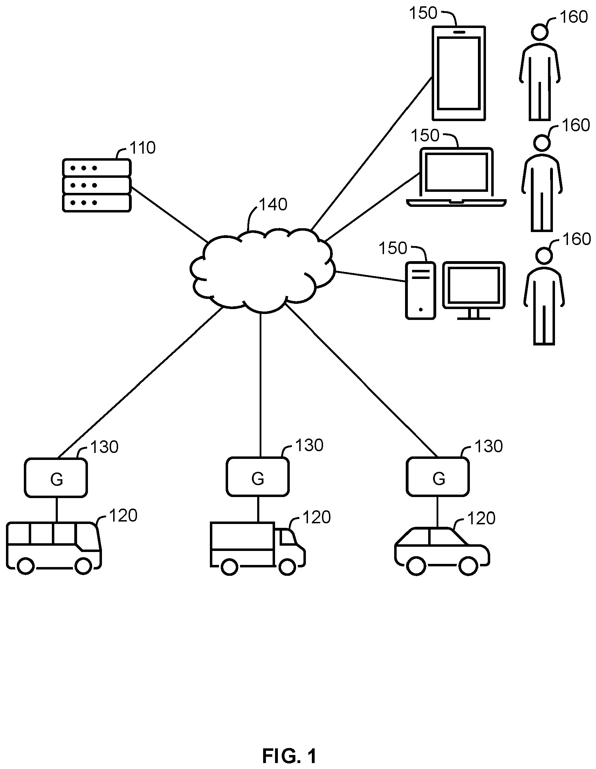

Referring now to FIG. 1, there is shown an example of a fleet management system 110 for managing a plurality of assets equipped with a plurality of telematics devices 130. Each of the telematics devices 130 is capable of collecting various data from the vehicles 120 (i.e., telematics data) and sharing the telematics data with the fleet management system 110. The fleet management system 110 may be remotely located from the telematics devices 130 and the vehicles 120.

The vehicles 120 may include any type of vehicle. For example, the vehicles 120 may include motor vehicles such as cars, trucks (e.g., pickup trucks, heavy-duty trucks such as class-8 vehicles, etc.), motorcycles, industrial vehicles (e.g., buses), and the like. Each motor vehicle may be a gas, diesel, electric, hybrid, and/or alternative fuel vehicle. Further, the vehicles 120 may include vehicles such as railed vehicles (e.g., trains, trams, and streetcars), watercraft (e.g., ships and recreational pleasure craft), aircraft (e.g., airplanes and helicopters), spacecraft, and the like. Each of the vehicles 120 may be equipped with one of the telematics devices 130.

Of course, in the context of the present disclosure, the vehicles 120 may generally be those that require engine oil for their operation - e.g., gas, diesel, and hybrid vehicles.

Further, it is noted that, while only three vehicles 120 having three telematics devices 130 are shown in the illustrated example, it will be appreciated that there may be any number of vehicles 120 and telematics devices 130. For example, the fleet management system 110 may manage hundreds, thousands, or even millions of vehicles 120 and telematics devices 130.

In some embodiments, the telematics devices 130 may be standalone devices that are removably installed in the vehicles 120 (e.g., aftermarket telematics devices). In other embodiments, the telematics devices 130 may be integrated components of the vehicles 120 (e.g., pre-installed by an OEM). As described herein, the telematics devices 130 may collect various telematics data and share the telematics data with the fleet management system 110. The telematics data may include any information, parameters, attributes, characteristics, and/or features associated with the vehicles 120. For example, the vehicle data may include, but is not limited to, location data, speed data, acceleration data, fluid level data (e.g., oil, coolant, and washer fluid), energy data (e.g., battery and/or fuel level), engine data, brake data, transmission data, odometer data, vehicle identifying data, error/diagnostic data, tire pressure data, seatbelt data, airbag data, or a combination thereof. In some embodiments, the telematics data may include information relating to the telematics devices 130 and/or other devices associated with or connected to the telematics devices 130. Regardless, it should be appreciated the telematics data is a form of electronic data that requires a computer (e.g., a processor such as those described herein) to transmit, receive, interpret, process, and/or store.

Once received, the fleet management system 110 may process the telematics data obtained from the telematics devices 130 to provide various analysis, predictions, reporting, etc. In some embodiments, the fleet management system 110 may process the telematics data to provide additional information about the vehicles 120, such as, but not limited to, trip distances and times, idling times, harsh braking and driving, usage rates, fuel economy, and the like. Various data analytics may be implemented to process the telematics data. The telematics data may then be used to manage various aspects of the vehicles 120, such as route planning, vehicle maintenance, driver compliance, asset utilization, fuel management, etc., which, in turn, may improve productivity, efficiency, safety, and/or sustainability of the vehicles 120.

A plurality of computing devices 150 may provide access to the fleet management system 110 to a plurality of users 160. The users 160 may use computing devices 150 to access or retrieve various telematics data collected and/or processed by the fleet management system 110 to manage and track the vehicles 120. As will be appreciated, the computing devices 150 may be any suitable computing devices. For example, the computing devices 150 may be any type of computers such as, but not limited to, personal computers, portable computers, wearable computers, workstations, desktops, laptops, smartphones, tablets, smartwatches, personal digital assistants (PDAs), mobile devices, and the like. The computing devices 150 may be remotely located from the fleet management system 110, telematic devices 130, and vehicles 120.

The fleet management system 110, telematics devices 130, and computing devices 150 may communicate through a network 140. The network 140 may comprise a plurality of networks and may be wireless, wired, or a combination thereof. As will be appreciated, the network 140 may employ any suitable communication protocol and may use any suitable communication medium. For example, the network 140 may comprise Wi-Fi™ networks, Ethernet networks, Bluetooth™ networks, near-field communication (NFC) networks, radio networks, cellular networks, and/or satellite networks. The network 140 may be public, private, or a combination thereof. For example, the network 140 may comprise local area networks (LANs), wide area networks (WANs), the internet, or a combination thereof. Of course, as will also be appreciated, the network 140 may also facilitate communication with other devices and/or systems that are not shown.

Further, the fleet management system 110 may be implemented using one or more computers. For example, the fleet management system 110 may be implemented using one or more computer servers. The servers may be distributed across a wide geographical area. In some embodiments, the fleet management system 110 may be implemented using a cloud computing platform, such as Google Cloud Platform™ and Amazon Web Services™. In other embodiments, the fleet management system 110 may be implemented using one or more dedicated computer servers. In a further embodiment, the fleet management system 110 may be implemented using a combination of a cloud computing platform and one or more dedicated computer servers.

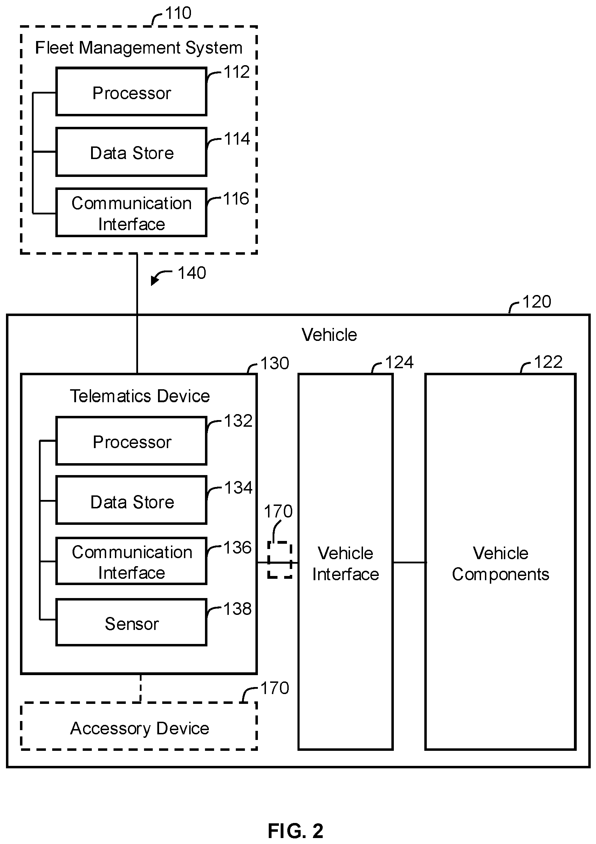

Referring now to FIG. 2, there is illustrated the fleet management system 110 in communication with one of the telematics devices 130 that is installed in one of the vehicles 120. As shown, the fleet management system 110 may include a processor 112, a data storage 114, and a communication interface 116, each of which may communicate with each other. The processor 112, the data storage 114, and the communication interface 116 may be combined into fewer components, divided into additional subcomponents, or a combination thereof. The components and/or subcomponents may not necessarily be distributed in proximity to one another and may instead be distributed across a wide geographical area.

The processor 112 may control the operation of the fleet management system 110. As will be appreciated, the processor 112 may be implemented using one or more suitable processing devices or systems. For example, the processor 112 may be implemented using central processing units (CPUs), graphics processing units (GPUs), field programmable gate arrays (FPGAs), application specific integrated circuits (ASICs), digital signal processors (DSPs), neural processing units (NPUs), quantum processing units (QPUs), microprocessors, controllers, and the like. The processor 112 may execute various instructions, programs, software, or a combination thereof stored on the data storage 114 to implement various methods described herein. For example, the processor 112 may process various telematics data collected by the fleet management system 110 from the telematics devices 130.

Various data for the fleet management system 110 may be stored on the data storage 114. The data storage 114 may be implemented using one or more suitable data storage devices or systems such as random-access memory (RAM), read only memory (ROM), flash memory, hard disk drives (HDDs), solid-state drives (SSDs), magnetic tape drives, optical disc drives, memory cards, and the like. The data storage 114 may include volatile memory, nonvolatile memory, or a combination thereof. Further, the data storage 114 may comprise non-transitory computer readable media. The data storage 114 may store various instructions, programs, and/or software that are executable by the processor 112 to implement various methods described herein. The data storage 114 may store various telematics data collected from the telematics devices 130 and/or processed by the processor 112.

The communication interface 116 may enable communication between the fleet management system 110 and other devices and/or systems, such as the telematics devices 130. The communication interface 116 may be implemented using any suitable communications devices and/or systems. For example, the communication interface 116 may comprise one or more various physical connectors, ports, or terminals such as universal serial bus (USB), ethernet, Thunderbolt, Firewire, serial advanced technology attachment (SATA), peripheral component interconnect (PCI), high-definition multimedia interface (HDMI), DisplayPort, and the like. As another example, the communication interface 116 may comprise one or more wireless interface components to connect to wireless networks such as Wi-Fi™, Bluetooth™, NFC, cellular, satellite, and the like. The communication interface 116 may enable various inputs and outputs to be received at and sent from the fleet management system 110. For example, the communication interface 116 may be used to telematics data from the telematics devices 130.

The telematics devices 130 also may include a processor 132, a data storage 134, and a communication interface 136. The telematics devices 130 may also comprise a sensor 138. Each of the components of the telematics devices 130 may communicate with each other and may be combined into fewer components or divided into additional subcomponents.

The processor 132 may control the operation of the telematics device 130. The processor 132 may be implemented using any suitable processing devices or systems, such as those described above in relation to the processor 112 of the fleet management system 110. The processor 132 may execute various instructions, programs, software, or a combination thereof stored on the data storage 134 to implement various methods described herein. For example, the processor 132 may process various telematics data obtained from vehicle components 122 and/or the sensor 138.

The data storage 134 may store various data for the telematics device 130. The data storage 134 may be any suitable data storage device or system, such as those described above in relation to the data storage 114 of the fleet management system 110. The data storage 134 may store various instructions, programs, software, or a combination thereof executable by the processor 132 to implement various methods described herein. As well, the data storage 134 may store various telematics data obtained from the vehicle components 122 and/or the sensor 138.

The communication interface 136 may enable communication between the telematics devices 130 and other devices or systems, such as the fleet management system 110 and the vehicle components 122. The communication interface 136 may comprise any suitable communication devices or systems, such as those described above in relation to the communication interface 116 of the fleet management system 110. The communication interface 136 may enable various inputs and outputs to be received at and sent from the telematics devices 130. For example, the communication interface 136 may be used to collect vehicle data from the vehicle components 122 and/or sensor 138, to send vehicle data to the fleet management system 110, etc.

The sensor 138 may detect and/or measure various environmental events, changes, etc. The sensor 138 may include any suitable sensing devices or systems, such as, but not limited to, location sensors, velocity sensors, acceleration sensors, orientation sensors, vibration sensors, proximity sensors, temperature sensors, humidity sensors, pressure sensors, optical sensors, audio sensors, and combinations thereof. When the telematics device 130 is installed in the vehicle 120, the sensor 138 may be used to collect telematics data that may not be obtainable from the vehicle components 122. For example, the sensor 138 may include a satellite navigation device such as a global positioning system (GPS) receiver that may measure the location of the vehicle 120. In some embodiments, the sensor 138 may comprise accelerometers, gyroscopes, magnetometers, inertial measurement units (IMUs), or the like that may measure the acceleration and/or orientation of the vehicle 120.

In some embodiments, the telematics devices 130 may operate in conjunction with one or more accessory devices 170 that are in communication therewith. The accessory devices 170 may include one or more expansion devices that may provide additional functionality to the telematics devices 130. For example, the accessory devices 170 may provide additional processing storage, communication, and/or sensing functionality through one or more additional processors, data storages, communication interfaces, and/or sensors (not pictured). The accessory devices 170 may also include adaptor devices that facilitate communication between the communication interface 136 and one or more vehicle interfaces 124, such as a cable harness. The one or more accessory devices 170 may be installed in the vehicle 120 along with the telematics devices 130.

As described herein, the telematics device 130 may be installed within the vehicle 120 removably or integrally. The vehicle 120 may include the vehicle components 122 and the one or more vehicle interfaces 124, which, as will be appreciated, may be combined into fewer components or divided into additional subcomponents. In some embodiments, the vehicle components 122 may comprise any subsystems, parts, subcomponents, or combinations thereof of the vehicle 120. For example, the vehicle components 122 may comprise powertrains, engines, transmissions, steering, braking, seating, batteries, doors, suspensions, etc. The telematics device 130 may obtain various telematics data from the vehicle components 122. For example, in some embodiments, the telematics device 130 may communicate with one or more electrical control units (ECUs) that control the vehicle components 122 or one or more internal sensors thereof.

The vehicle interface 124 may facilitate communication between the vehicle components 122 and other devices or systems. As well, the vehicle interface 124 may comprise any suitable communication devices or systems. For example, the vehicle interface 124 may include an on-board diagnostics (OBD-II) port and/or controller area network (CAN) bus port. The vehicle interface 124 may be used by the telematics device 130 to obtain telematics data from the vehicle components 122. For example, the communication interface 136 may be connected to the vehicle interface 124 to communicate with the vehicle components 122. In some embodiments, the one or more accessory devices 170 (e.g., a wire harness) may provide the connection between the communication interface 136 and the vehicle interface 124.

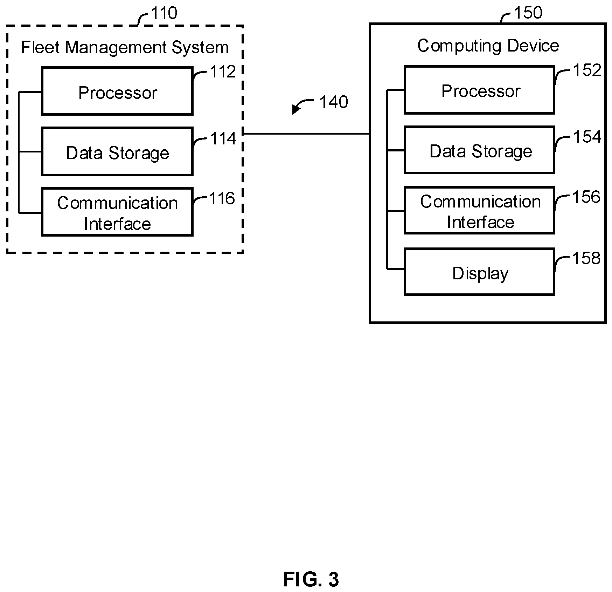

Referring now to FIG. 3, there is shown the fleet management system 110 in communication with the computing devices 150. As shown, the computing device 150 may also include a processor 152, a data storage 154, and a communication interface 156. As well, the computing device 150 may include a display 158. Each of the components of the computing device 150 may be communicate with each other and may be combined into fewer components or divided into additional subcomponents.

The processor 152 may control the operation of the computing device 150. The processor 152 may be implemented using any suitable processing devices or systems, such as those described above in relation to the processor 112 of the fleet management system 110. The processor 152 may execute various instructions, programs, software, or a combination thereof stored on the data storage 154 to implement various methods described herein. For example, the processor 152 may process various telematics data received from the fleet management system 110, the telematics devices 130, or a combination thereof.

The data storage 154 may store various data for the computing device 150. The data storage 150 may be any suitable data storage device or system, such as those described above in relation to the data storage 114 of the fleet management system 110. The data storage 154 may store various instructions, programs, software, or a combination thereof executable by the processor 152 to implement various methods described herein. As well, the data storage 154 may store various telematics data received from the fleet management system 110, the telematics devices 130, or a combination thereof.

The communication interface 156 may enable communication between the computing device 150 and other devices or systems, such as the fleet management system 110. The communication interface 156 may be any suitable communication device or system, such as those described above in relation to the communication interface 116 of the fleet management system 110. The communication interface 156 may enable various inputs and outputs to be received at and sent from the computing device 150. For example, the communication interface 156 may be used to retrieve telematics data the fleet management system 110.

The displays 158 may visually present various data for the computing device 150. The displays 158 may be implemented using any suitable display devices or systems, such as, but not limited to, light-emitting diode (LED) displays, liquid crystal displays (LCD), electroluminescent displays (ELDs), plasma displays, quantum dot displays, cathode ray tube (CRT) displays, and the like. The display 158 may be an integrated component that is integral with the computing device 150 or a standalone device that is removable connected to the computing device 150. The display 158 may display various visual representations of the telematics data.

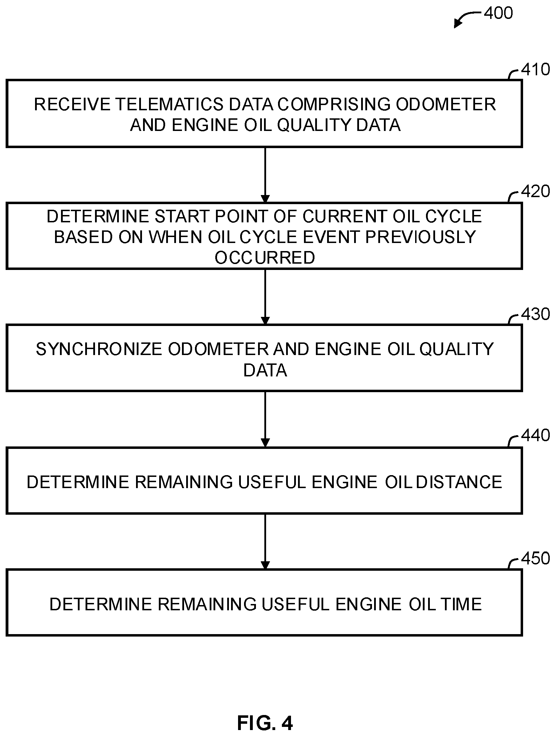

Referring now to FIG. 4, there is shown an example of a method for estimating a remaining useful life of vehicle engine oil (400) according to an embodiment of the present disclosure. As shown, the method 400 may comprise operating at least one processor to: receive telematics data originating from a plurality of vehicles, the telematics data comprising odometer data and engine oil quality data associated with each of the plurality of vehicles (410); determine a start point of a current oil cycle based on when an oil cycle event previously occurred by identifying within the engine oil quality data: a first series of engine oil quality datapoints, and a second series of engine oil quality datapoint immediately following the first series, a difference between an final datapoint of the first series and an initial datapoint of the second series being greater than a predetermined threshold (420); synchronize the odometer data and the engine oil quality data by associating odometer datapoints and engine oil quality datapoints reported within a same time period (430); determine a remaining useful vehicle engine oil distance by applying to synchronized odometer and engine oil quality data reported during the current oil cycle a regression model (440); and determine a remaining useful vehicle engine oil time by converting the remaining useful vehicle engine oil distance thereto based on an average difference in odometer datapoints reported during a selected time period (450), whereby the remaining useful life of vehicle engine oil is estimated based on vehicle operational factors associated with the current oil cycle, independently from those of a previous oil cycle.

The method 400 may be implemented using any suitable combination of hardware and software, such as those described in reference to FIG. 1 to FIG. 3, due at least in part to the nature of telematics data and, in some implementations, the large amounts of telematics data being processed. For example, one or more operations (e.g., operations 410, 420, 430, 440, and/or 450) of the method 400 may be implemented at the fleet management system (e.g., by the processor 112 executing instructions stored on the data storage 114), at the telematics device 130 (e.g., by the processor 132 executing instructions stored on the data storage 134), at the computing devices 150 (e.g., by the processor 152 executing instructions stored on the data storage 154), or a combination thereof.

In embodiments where at least a portion of the method 400 is implemented by a fleet management system (e.g., the fleet management system 110), less processing may be executed by telematics devices (e.g., the telematics devices 130) and/or other computing devices (e.g., the computing devices 150). As a result, the hardware complexity of the telematics devices and/or the other computing devices may be reduced, which, in turn, may reduce the costs associated therewith. As well, it may also in some cases be easier to update and/or modify software running on a fleet management system as compared to telematics devices and/or other computing devices. On the other hand, in embodiments where at least a portion of the method 400 is implemented by telematics devices, less data may be transmitted to a fleet management system and/or other computing devices, thereby reducing network usage and network bandwidth. As will be appreciated, in such implementations, usage costs associated with network usage may in turn also be reduced. Thus, the method 400 may be implemented in a variety of ways and each implementation may have advantages associated therewith.

At operation 410, telematics data associated with a plurality of vehicles may be received. The telematics data may be obtained from the plurality of vehicles using, for example, one or more of the systems outlined in FIG. 1 to FIG. 3. For example, the telematics device 130 (e.g., the processor 132) may receive telematics data from the sensor 138, vehicle components 122, or a combination thereof. Alternatively, or additionally, the fleet management system 110 (e.g., the processor 112) may receive telematics data from the telematics device 130. Additionally, or alternatively, the computing device 150 (e.g., the processor 152) may receive telematics data from the telematics device 130 and/or the fleet management system 110. Additionally, or alternatively, the telematics device 130, the fleet management system 110, and/or the computing device 150 may receive telematics data from one or more data storages (e.g., one or more of the data storages 114, 134, 154).

In the systems and methods of the present disclosure, the telematics data associated with the plurality of vehicles may generally include engine data - i.e., telematics data relating to one or more aspects of the engine of the vehicles. The engine data may comprise at least odometer data and engine oil quality data.

Odometer data may indicate an odometer reading of the vehicle at a given time. As will be appreciated, an odometer measures the total distance travelled by a vehicle, typically based on a number of wheel rotations performed thereby. The odometer data may therefore indicate a total distance travelled by the vehicle at a given time. The units used to quantify the distance travelled by the vehicle may vary depending on, for example, where the vehicle is expected to operate. For example, an odometer of a vehicle manufactured and/or sold in a market such as the US may measure distance in miles, while an odometer of a vehicle manufactured and/or sold in a market such as Canada may measure distance in kilometers.

The engine oil quality data may indicate an estimation of the engine oil quality (i.e., a remaining useful life of the engine oil) at a given time. The engine oil quality data may be generated using a proprietary algorithm of the vehicle OEM, as described herein. The engine oil data may be represented as a percentage (i.e., a percentage of useful life remaining), a time (i.e., a time until the engine oil is no longer useful), a distance (i.e., a distance until the engine oil is no longer useful), or the like depending, for example, on the particular algorithm employed by the vehicle OEM.

As will be described herein, the odometer data and the engine oil quality data may be used to determine a more accurate estimation the remaining useful life of vehicle engine oil of a vehicle (e.g., relative to at least that determined by the vehicle OEM). Of course, the telematics data may include additional types of data. In one example, in some embodiments, the telematics data may further comprise vehicle identifying data (e.g., vehicle identification numbers, or "VINs"), which, as will be described herein, may be used during the estimation of the remaining useful life of engine oil.

Further, in some embodiments, the telematics data may be preprocessed prior to and/or subsequently to being received. For example, the telematics data may be received in one or more various formats, standards, or protocols. In some cases, it may be beneficial to reformat the telematics data prior to use in the systems and methods of the present disclosure. As a further example, the telematics data may include datapoints reported at irregular frequencies and/or that correspond to mismatched points in time. In such cases, the telematics data may be interpolated so that the datapoints in each time series correspond to successive and/or equally spaced points in time. As a yet further example, and as will be described herein, the telematics data may be curve-logged telematics data, which may result in a reduced number of received datapoints. In such implementations, the reduced number of datapoints may be interpolated to provide a fulsome dataset.

At operation 420 of the method 400, a start point of a current oil cycle may be determined based on when an oil cycle event previously occurred.

As used herein, an "oil cycle event" generally refers to a significant change in engine oil quality (i.e., remaining useful life of engine oil). The occurrence of an oil cycle event may be determined by identifying portions of the engine oil quality data that satisfy one or more predetermined conditions. For example, in some embodiments, the one or more predetermined conditions may include a significant change in engine oil quality. In such embodiments, the significant change may be a change in engine oil quality greater than a predetermined threshold. The predetermined threshold may correspond to a change in engine oil quality that is greater than or equal to 10%, 20%, 30%, 40%, 50%, or greater, or less, or any percentage therebetween. The particular predetermined threshold may be selected based on a number of factors such as, but not limited to, the type of vehicle, the type of engine oil (e.g., synthetic engine oil vs. semi-synthetic engine oil vs. conventional engine oil), the amount of noise in the engine oil quality data, etc.

Further, it is noted that the significant change in engine oil quality may be a positive change (i.e., a significant increase in engine oil quality) or a negative change (i.e., a significant decrease in engine oil quality). An increase in engine oil quality may indicate that an oil change event occurred (e.g., a vehicle operator changed, or replaced, the engine oil of the vehicle) while a decrease in engine oil quality may indicate that an oil drain event occurred (e.g., a vehicle operator drained the engine oil but did not replace with new engine oil). As will be appreciated, an oil drain event may also, or instead, indicate that there is an issue with the vehicle.

As used herein, the term "oil cycle" refers to a period of time that a vehicle operates prior to, after, and/or between oil cycle events. For example, an oil cycle may refer to a period of time that a vehicle operated between two oil cycle events (e.g., oil change events). As another example, an oil cycle may refer to a period of time that a vehicle operates after a most recent oil cycle event (i.e., a "current oil cycle"). As a yet further example, an oil cycle may refer to a period of time that a vehicle operated before a first oil cycle event (e.g., a first oil change after manufacture of the vehicle).

Thus, a start point of a current oil cycle of a vehicle may correspond to when a most recent previous oil cycle event occurred. The determination of the start point of the current oil cycle may be implemented using any suitable hardware and/or software. For example, one or more processors (e.g., one or more of the processors 112, 132, 152) may be used to identify when a previous oil cycle event occurred based on the satisfying of one or more predetermined conditions of the engine oil quality data, as will be described below.

As indicated above, oil cycle events may be identified based on significant changes in engine oil quality. In more detail, as described herein, engine oil quality data may include a plurality of datapoints indicating the engine oil quality of a vehicle at a given time. A significant difference between two consecutive datapoints of the engine oil quality data may indicate an oil cycle event. As described above, the difference may be greater than a predetermined threshold to indicate the oil cycle event (e.g., a difference greater than or equal to 10%, 20%, 30%, 40%, 50%, or greater, or less, or any percentage therebetween) and may be a positive change and/or a negative change.

However, the engine oil quality data may in some cases be "noisy" (e.g., include random increases and decreases therein). Such noise may cause a significant difference between two engine oil cycle datapoints, which may, in turn, be incorrectly identified as an oil cycle event (i.e., a false positive). In such cases, it may therefore be useful to use, for example, more than two engine oil quality data points to identify oil cycle events, thereby mitigating any effect noise may have on the identification of oil cycle events.

In more detail, when a previous oil cycle event occurred may be determined by identifying within the engine oil quality data a first series of engine oil quality datapoints and a second series of engine oil quality datapoints, wherein a difference between a final datapoint of the first series and an initial datapoint of the second series is greater than a predetermined threshold (e.g., indicating a significant change in engine oil quality). In such implementations, the data points of the first and second series may be considered when identifying when the previous oil cycle occurred to reduce the likelihood of a false-positive caused by noisy data.

The first and second series of engine oil quality datapoints may each independently comprise a plurality of datapoints. For example, in some embodiments, each of the first and second series may include 10, 20, 30, 40, or 50 datapoints, or more, or fewer, or any number of datapoints therebetween.

In some embodiments, the satisfying of one or more additional predetermined conditions by the first and second series of engine oil quality datapoints may be identified to provide additional information about the oil cycle event, to further reduce the likelihood of a false positive, etc. For example, an oil cycle event may be identified when an initial datapoint of the first series is greater than the final datapoint thereof, and the initial datapoint of the second series is greater than a final datapoint of thereof, as the satisfying of such predetermined conditions may indicate that engine oil quality is decreasing over time, which is to be expected during normal operation of a vehicle, and may, in turn, indicate that the engine oil quality data may not be particularly noisy.

As a further example, in some embodiments, an oil cycle event may be identified when each datapoint of the first series and the second series decreases relative to an immediately preceding datapoint thereof. Additionally, or alternatively, an oil cycle event may be identified when each of the datapoints of the second series are greater than or less than each of the datapoints of the first series. The satisfying of such predetermined conditions may further demonstrate that that engine oil quality is expectedly decreasing over time and/or that a significant change was due to an oil cycle event, rather than random noise, which in turn may indicate that the engine oil quality data may not be noisy However, as will be appreciated, engine oil quality data may still have a minor amount of noise. For example, datapoints in a series (e.g., the first series and/or the second series) may increase or decrease relative to each other at random but less than a predetermined threshold for determining whether an oil cycle event occurred, as described above. In such cases, it may be useful to account for such noise when identifying when an oil cycle event occurred. For instance, in some embodiments, an oil cycle event may be identified when each datapoint of the first series and the second series is greater than or less than an immediately preceding datapoint within a predetermined threshold (e.g., the predetermined threshold used to identify the significant change in engine oil quality described above), or is equal to the immediately preceding datapoint.

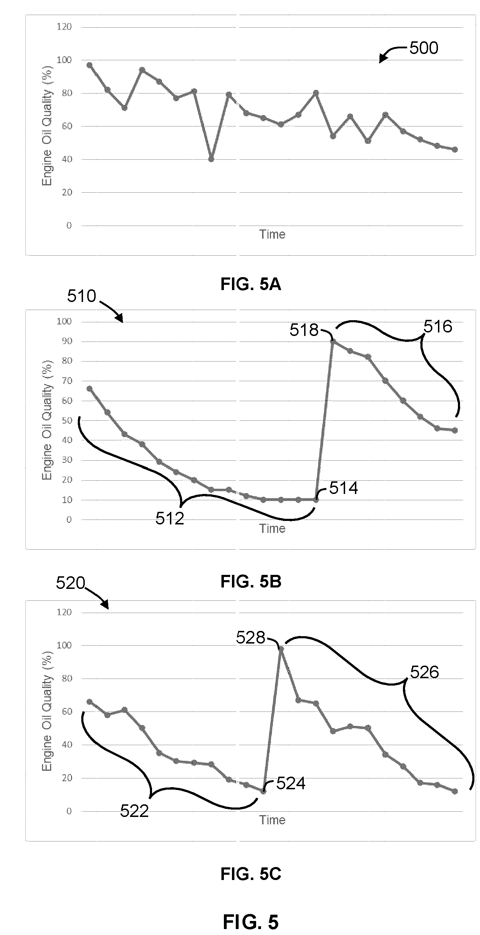

For additional clarity, FIG. 5 shows a plurality of examples of engine oil quality data, each obtained from a different vehicle, changing over time. FIG. 5A depicts a first example engine oil quality data 500. In that example, the engine oil quality data 500 is "noisy" in that there are substantial increases and decreases between engine oil quality datapoints that, if considered alone, may indicate that an oil cycle event occurred. However, as shown, such increases and decreases may not be indicative of an oil cycle event. Thus, as described above, it may be useful to consider engine oil quality datapoints in addition to those between which there is a significant difference (e.g., the first and second series of datapoints and features thereof).

As another example, FIG. 5B shows a second example of engine oil quality data 510, which includes a first series of engine oil quality datapoints 512 and a second series of engine oil quality datapoints 516 immediately following the first series 512. As shown, an initial datapoint 518 of the second series 516 is greater than a final datapoint 514 of the first series 512, each datapoint of the first series 512 and the second series 516 decreases relative to a previous datapoint, and each datapoint of the second series 516 is greater than each datapoint of the first series 512. As described above, the satisfying of one or more of such predetermined conditions by the engine oil quality data 510 may be indicative of an oil cycle event occurring.

Another example of engine oil quality data 520 is shown in FIG. 5C, which includes a first series of engine oil quality datapoints 522 and a second series of engine oil quality datapoints 526. As shown, an initial datapoint 528 of the second series 526 is greater than a final datapoint 524 of the first series 522 and each datapoint of the second series 526 is greater than each datapoint of the first series 522. However, each datapoint of the first series 522 and the second series 526 does not decrease relative to an immediately preceding datapoint thereof. Thus, it may be useful to instead determine whether each datapoint of the first series 522 and the second series 526 is greater than or less than an immediately preceding datapoint within a predetermined threshold, or is equal to the immediately preceding datapoint, as described above.

In light of the above, it may therefore be useful to identify whether the engine oil quality data satisfies a plurality of predetermined conditions that may be indicative of an oil cycle event. For example, in some embodiments, when an oil cycle event previously occurred may be determined by identifying within the engine oil quality data a first series of engine oil quality datapoints, a second series of engine oil quality datapoints, a difference between a final datapoint of the first series and an initial datapoint of the second series being greater than a predetermined threshold, each datapoint of the second series being greater or less than each datapoint of the first series, each datapoint of the first series and the second series decreasing relative to an immediately preceding datapoint, and/or, if each datapoint of the first series and the second series does not decrease relative to an immediately preceding datapoint, each datapoint of the first series and the second series being greater or less than an immediately preceding datapoint within a predetermined threshold or equal to the immediately preceding datapoint.

Alternatively, in some embodiments, if the first series and second series are not present within the engine oil quality data (e.g., there has been no previous oil cycle event), the at least one processor may be operable to determine the start point of the current oil cycle to be a first reported engine oil quality datapoint.

In some embodiments, the engine oil quality data used to identify the previously occurring oil cycle event may be all available engine oil quality data obtained from the respective vehicle (e.g., engine oil quality data reported when the vehicle first started to operate, engine oil quality data obtained after initial installation of a telematics device, etc.). In another embodiment, the engine oil quality data may be that obtained from within a selected reporting period. That is, in such embodiments, engine oil quality data reported within a selected time period may be used to identify when an oil cycle event previously occurred. For example, in such embodiments, the determining of the start point of the current oil cycle may comprise: selecting engine oil quality data reported within a selected reporting period; and identifying within the engine oil quality data reported within the selected reporting period the first series and the second series.

The selected reporting period may be any suitable time period. For instance, the selected reporting period may be chosen based on when and/or how often the systems and methods of the present disclosure are implemented. For example, if the systems and methods of the present disclosure are implemented daily at a predetermined time, it may be useful for the selected reporting period to be a previous 24-hour period so that any oil cycle events occurring after the previous implementation of the systems and methods of the present disclosure may be identified. In another example, the selected reporting period may be chosen such that a minimum amount of engine oil quality datapoints are reported and available for use to identify any oil cycle events. In more detail, as described above, determining when an oil cycle event previously occurred generally involves identifying a first and second series of engine oil datapoints as well as whether those series satisfy one or more predetermined conditions. As identifying the first and second series may require a plurality of engine oil quality datapoints, it may therefore be useful to choose for the selected reporting period a time period in which a plurality of data points may be reported. For example, the selected reporting period may be chosen such that at least 10, 20, 30, 40, or 50 datapoints, or more, or fewer, or any number therebetween are received therewithin.

Referring back to FIG. 4, at operation 430, the odometer data and the engine oil quality data may be synchronized by associating odometer datapoints and engine oil quality datapoints reported within a same time period. In more detail, odometer data and engine oil quality data may be associated with each other such that engine oil quality at a particular odometer datapoint, or vice versa, may be determined.

As indicated herein, different types of telematics data may be produced and/or reported at different frequencies. For example, a vehicle may produce the odometer data and the engine oil quality data at different frequencies such that, while the odometer data may include an odometer datapoint of the vehicle at a particular time, the engine oil quality data may not include an engine oil quality datapoint for that time. As another example, and as indicated above, the telematics data obtained from a vehicle may be "curve-logged" in that a dataset simplification algorithm may be employed to reduce the amount of data reported (e.g., via the telematics device 130), based on whether the telematics data produced by the vehicle may be useful to, for instance, a fleet management system (e.g., the fleet management system 110). In that example, due to the curve logging, odometer data and engine oil quality data may effectively be reported at different frequencies.

Thus, it may be useful to synchronize the odometer data and the engine oil quality data based on a time period within which the data was reported, rather than a specific time. For example, in some embodiments, the odometer data and the engine oil quality data may be synchronized by associating odometer datapoints with engine oil quality datapoints reported within a minute, 5 minutes, 10 minutes, an hour, 1.5 hours, or more, or less, or any time period therebetween, of each other. The particular time period within which the odometer data and the engine oil quality data may be reported to be synchronized may be selected based on, for example, the frequency at which a vehicle produces the data, the frequency at which a telematics device reports the data, etc.

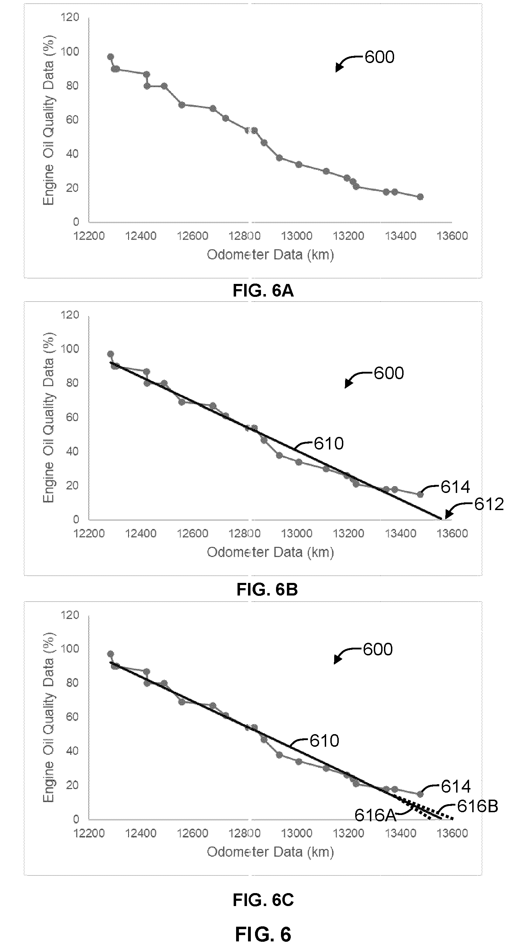

Once the odometer data and the engine oil quality data are synchronized, the data may be analyzed using one or more statistical models, as will be described herein. For example, the odometer data and the engine oil quality data may be associated with each other such that the synchronized data may be plotted (e.g., odometer datapoints vs. engine oil quality datapoints) for further analysis. For additional clarity, an example of synchronized odometer and engine oil quality data 600 is illustrated in FIG. 6A.

As will be appreciated, the synchronizing of the odometer data and the engine oil quality data may be performed using any suitable hardware and/or software. For example, one or more of the processors 112, 132, 152 may identify when odometer and engine oil quality datapoints were received, and synchronize datapoints reported within a same time period.

Referring again to FIG. 4, at operation 440, a remaining useful vehicle engine oil distance may be determined by applying to synchronized odometer and engine oil quality data reported during the current oil cycle a regression model.

As used herein, the term "remaining useful vehicle engine oil distance" generally refers to a distance a vehicle may drive until the engine oil thereof is no longer useful (e.g., the engine oil quality approaches or reaches zero). As indicated above, the remaining useful vehicle engine oil may be determined using synchronized odometer and engine oil quality data reported during the current oil cycle (i.e., the data reported after the occurrence of the most recent previous oil cycle event).

In more detail, a statistical model such as a regression model may be applied to synchronized odometer and engine oil quality data reported during the current oil cycle to determine a distance that the vehicle may travel before the engine oil thereof should be replaced (i.e., the remaining useful engine oil distance). The regression model applied to the synchronized odometer and engine oil quality data is not particularly limited and may comprise, for example, a linear regression model, a polynomial regression model, a linear degradation model, an exponential degradation model, and/or the like.

In some embodiments, the particular regression model applied to the synchronized odometer and engine oil quality data may be selected based on one or more vehicle parameters of the one or more of the plurality of vehicles. For example, as described herein, the telematics data may comprise vehicle identifying information such as, but not limited to, VINs. As will be appreciated, different types of vehicles (e.g., with respect to engine type, engine size, make, model, etc.) may degrade engine oil at different rates. It may therefore be useful to select a regression model based on the type of vehicle for which the remaining useful life of vehicle engine oil is to be estimated, as a particular regression model may be better suited for predicting the remaining useful life of engine oil of a particular vehicle type due to the rate of oil degradation thereof. Thus, in some embodiments, the determining of the remaining useful vehicle engine oil distance may comprise identifying a vehicle type of each of the one or more of the plurality of vehicles based on the vehicle identifying information; selecting, for each of the one or more of the plurality of vehicles, a type of regression model based on the vehicle type of each thereof; and applying the regression model to the synchronized odometer and EOLR data reported during the current oil cycle. The vehicle type for which the regression model may be selected may be a classification of vehicle (e.g., passenger vehicles vs industrial vehicles such as heavy-duty trucks), the make of the vehicle, the model of the vehicle, etc.

In some embodiments, the determining of the remaining useful vehicle engine oil distance may comprise: determining from the regression model at least a rate of degradation of the engine oil quality of the vehicle; determining, based on the rate of degradation, an odometer reading at which the engine oil quality indicates that the engine oil should be changed; and determining the remaining useful vehicle engine oil distance based on a difference between a most recent odometer datapoint and the odometer reading at which the engine oil quality indicates engine oil should be changed. In such embodiments, the rate of degradation may correspond to, for example, a slope of a line fit to the synchronized data, depending on the type of regression model applied. The odometer datapoint at which the engine oil quality indicates that the engine oil of the vehicle should be changed may correspond to, for example, an odometer datapoint at which the engine oil quality is predicted to be zero. The difference between that odometer datapoint and a most recent odometer datapoint (e.g., a most recently reported odometer datapoint), which as will be appreciated both correspond to a distance travelled by the vehicle, may therefore indicate a distance that the vehicle may travel before the engine oil should be changed.

For additional clarity, FIG. 6B shows the synchronized odometer data and engine oil quality data 600 illustrated in FIG. 6A having a linear degradation model applied thereto 610. As shown, the linear degradation model 610 may be used to indicate an odometer reading 612 at which the engine oil quality is predicted to be zero. As described above, a difference between that odometer datapoint 612 and a most recent odometer datapoint 614 may indicate a distance that a vehicle may travel before the engine oil should be changed.

In some embodiments, it may be useful to estimate error, or confidence, associated with the determined remaining useful engine oil distance. In such embodiments, the remaining useful engine oil distance, and in turn a remaining useful engine oil time determined therefrom as will be described herein, may comprise a range based on the confidence bounds of the rate of degradation. For example, the remaining useful engine oil distance may include an estimated range of distances that the vehicle may travel before the engine oil thereof should be changed.

For additional clarity, FIG. 6C shows the synchronized odometer data and engine oil quality data having the linear degradation model 610 applied thereto illustrated in FIG. 6B with confidence bounds 616A, 616B determined therefor. As described above, the confidence bounds 616A, 616B may be used to identify a range of distances that the vehicle may travel before the engine oil should be changed. In this in this case, the difference between the odometer datapoints at which the each of the confidence bounds 616A, 616B indicates the engine oil quality will become zero and the most recent odometer datapoint 614 may define the bounds of the range.

The confidence bounds of the useful remaining engine oil distance may be determined using any suitable technique. For example, the confidence bounds of the rate of degradation may be determined based on parameters thereof (e.g., residual standard deviation) and a range of remaining useful engine oil distance may be determined therefrom (e.g., by one or more of the processors 112, 132, 152).

However, as indicated above, the number of synchronized odometer and engine oil quality datapoints available for analysis (e.g., for application of a regression model thereto) may vary depending on, for example, the frequency at which the odometer data and engine oil quality data is produced (e.g., by the vehicle) and/or reported (e.g., due to curve logging of the telematics data). As will be appreciated, variations in the amount of synchronized odometer and engine oil quality datapoints available for analysis may affect the determination of certain parameters by the regression model applied thereto. For example, in some embodiments, the determination of the confidence bounds of the rate of degradation may comprise operating the at least one processor to determine the confidence bounds based at least in part on a residual standard deviation of the rate of degradation. However, residual standard deviation may be inversely proportional to a number of datapoints to which a regression model may be applied. As a result, it may be the case that, using only standard deviation may not determine confidence bounds consistently for synchronized odometer and engine oil quality data having differing numbers of datapoints - e.g., for different vehicles, for different oil cycles of a vehicle, etc.

Thus, using only residual standard deviation may not produce confidence bounds consistently for synchronized odometer and engine oil quality data having differing numbers of datapoints. It may therefore be useful to account for any variances resulting from differing numbers of synchronized odometer and engine oil quality datapoints using a confidence coefficient. For example, the determining of the remaining useful engine oil distance may further comprise operating the at least one processor to determine confidence bounds for the rate of degradation based on a residual standard deviation thereof and a confidence coefficient.

The confidence coefficient may be used to minimize the effect that the number of datapoints of the synchronized odometer and engine oil quality data has on the residual standard error such that the confidence bounds are generated consistently each time the systems and methods of the present disclosure are implemented, regardless of how many synchronized odometer and engine oil quality datapoints the regression model is applied to. The confidence coefficient may be generated using, for example, identified previous oil cycles for the vehicle (e.g., ground truth data).

As one example, a confidence coefficient may be generated using one or more parameters determined by applying a regression model to synchronized odometer and engine oil quality data of a previous oil cycle. In that example, from the applied regression model, a mean rate of degradation of the synchronized odometer and engine oil quality data of the previous oil cycle and a residual standard deviation thereof may be determined. Because the synchronized odometer and engine oil quality data are from the previous oil cycle, the true rate of degradation thereof may also be determined. With the true rate of degradation, the mean rate of degradation, and the residual standard deviation, the confidence coefficient may be determined using, for example, the following formula for determining confidence bounds of the rate of degradation:

$$\beta_T \in \beta_m \pm (C_c \times \sigma_R)$$

wherein, $\beta_T$ is the true rate of degradation, $\beta_M$ is the mean rate of degradation, $C_C$ is the confidence coefficient, and $\sigma_R$ is the residual standard deviation. As will be appreciated, using the above formula, the confidence coefficient may be determined as shown below:

$$C_c = \frac{|\beta_T - \beta_m|}{\sigma_R}$$

However, confidence coefficient may also vary somewhat based on the amount of datapoints to which the regression model was applied. Thus, it may be useful to determine an average confidence coefficient to minimize such variations. For example, an average confidence coefficient may be generated by applying the regression model to different numbers of synchronized odometer and engine oil quality datapoints of the previous oil cycle to determine a confidence coefficient for each of the different numbers of datapoints. The plurality of determined confidence coefficients may then be averaged to provide the averaged confidence coefficient.

It may in some cases be useful to optimize the generation of the confidence coefficient such that the confidence bounds generated in part therefrom have a minimal spread (i.e., a minimum distance between upper and lower confidence bounds). As will be appreciated, by generating confidence bounds having a minimal spread, a more precise range for the remaining useful engine oil life distance may be determined. In some embodiments, the optimization of the generation of the confidence coefficient and, in turn, the confidence bounds, may comprise generating a range of confidence coefficients (e.g., averaged confidence coefficients), by applying at different points of the synchronized odometer and engine oil quality data the regression model. The range of confidence coefficients may then be used to determine an optimized final confidence coefficient from which optimized confidence bounds may be generated. For example, the final confidence coefficient may be selected from within the range of confidence coefficients. As another example, the range of confidence coefficients may be aggregated, averaged, or the like to identify the final confidence coefficient. As another example, the final confidence coefficient may be generated via hyperparameter optimization using the range of confidence coefficients.

In some embodiments, the confidence coefficient (e.g., the averaged confidence coefficient, the final confidence coefficient, etc.) may be generated on a per-vehicle basis (i.e., a confidence coefficient is generated for each vehicle). In another embodiment, the confidence coefficient may be generated for use in the determination of a range of remaining useful vehicle engine oil life estimations (e.g., remaining useful vehicle engine oil distance and/or time) for a plurality of vehicles. As will be appreciated, the generation of a confidence coefficient for each vehicle may be a particularly processor-intensive operation. Thus, to increase the efficiency of the systems and methods of the present disclosure, it may in some cases be useful to generate a confidence coefficient that that may be used for a plurality of vehicles. The plurality of vehicles may be vehicles of the same type (e.g., passenger vehicles, industrial vehicles, etc.), vehicles of the same make and/or model, vehicles having the same or similar (e.g., within about 5%, 10%, etc.) amount of telematics data available (e.g., the number of odometer and/or engine oil quality datapoints reported), vehicles having the same or similar the amount of synchronized odometer and engine oil quality datapoints available for analysis, etc.

For example, in an embodiment, the determination of the confidence coefficient may comprise operating the at least one processor to select a confidence coefficient based on one or more vehicle parameters. Thus, in such embodiments, the confidence coefficient may be pre-generated. The one or more vehicle parameters upon which the selection of the confidence coefficient is based may be, for example, any of those described above such as, but not limited to, vehicle type, vehicle make (i.e., OEM of the vehicle), vehicle model, amount of telematics data reported, number of synchronized odometer and engine oil quality data points, etc.

Referring back to FIG. 4, at 450 it is shown that at a remaining useful vehicle engine oil time may be determined by converting the remaining useful vehicle engine oil distance thereto based on an average difference in odometer datapoints reported during a selected time period. As described above, the remaining useful vehicle engine oil time may refer to an amount of time until a vehicle's engine oil is no longer useful or, put differently, should be changed. Also, as indicated above, remaining useful life of vehicle engine oil time may refer to a single time (e.g., 3 days until the vehicle engine oil should be changed), or a range of times (e.g., 2 weeks to 3 weeks until the vehicle engine oil should be changed). The remaining useful engine oil time may be determined in terms of any desired units. For example, the remaining useful engine oil time may be determined in terms of hours, days, weeks, months, etc. The particular units used may be selected or changed based on the amount useful engine oil time remaining (e.g., 1 week vs. 168 hrs).

The remaining useful engine oil distance may be converted to the remaining useful engine oil time based on the average difference in odometer datapoints reported during a selected time period. In some embodiments, the average difference between odometer datapoints may be, for example, an average daily, weekly, monthly, etc. difference in the odometer datapoints. The particular average difference in odometer datapoints may be selected based on, for example, how long the vehicle has been on the current oil cycle, the amount of odometer data received during the current oil cycle, an overall change in odometer datapoints from the beginning of the current oil cycle, how long the vehicle has been operating, the total amount of odometer data available, the frequency at which the odometer data is reported, etc.

The average difference in odometer datapoints may be between those reported over a selected time period within the current oil cycle, or not. As will be appreciated, the average difference in odometer datapoints may be an indication of the distances travelled by the vehicle each day, week, month, etc. during the selected time period. Such distances travelled may not be affected by the occurrence of an oil cycle event or events. The particular time period from which the odometer datapoints are reported may be any suitable time period. For example, in some embodiments, the time period may be an amount of time elapsed from the start point of the current oil cycle. In another embodiment, the time period may be a predetermined time period such as, but not limited to, 1 day, 1 week, 4 weeks, 6 weeks, etc. As will be appreciated, such embodiments may be less processor-intensive if the amount of time elapsed from the start point of the current oil cycle is particularly long.

As will be appreciated, the remaining useful engine oil time may be determined, for example, by operating at least one processor (e.g., one or more of the processors 112, 132, 152) to divide the remaining useful engine oil distance by the average difference in odometer datapoints over the selected time period.

In some embodiments, the systems and methods of the present disclosure may be implemented repeatedly to estimate the remaining useful engine oil life as time progresses, as the vehicle operates, etc. so that newly received telematics data (e.g., odometer data and engine oil quality data) may be factored into the estimation. In such embodiments, the systems and methods may repeat the estimation of the remaining useful life of the vehicle engine oil at a predetermined cadence. That is, the systems and methods of the present disclosure may be implemented at regular time intervals. For example, the systems and methods of the present disclosure may be implemented once a day, twice a day, once a week, twice a week, or any other time interval. In some embodiments, the systems and methods of the present disclosure may be implemented when a predetermined amount of telematics data has been received and/or upon the synchronization of a predetermined amount of odometer and engine oil quality datapoints. For example, in such embodiments, the systems and methods of the present disclosure may be implemented upon receipt, and/or synchronization, of at least 5, 10, 15, 20, or more, or fewer odometer and/or engine oil datapoints.

In light of the above, the remaining useful life of the vehicle engine oil may be estimated based on vehicle operational factors associated with the current oil cycle, independently from those of a previous oil cycle. That is, the estimation of the remaining useful engine oil life may be based on vehicle operational factors such as oil type, driving habits, driving conditions, and the like that were used, or occurred, during the current oil cycle, as the estimation is based on odometer data (which may indicate, for example, driving habits) and engine oil quality data (which may indicate, for example, engine oil type used, weather conditions the vehicle has operated in, etc.) reported therein. Thus, the systems and methods of the present disclosure may more accurately estimate remaining useful vehicle engine oil life than, for example, the proprietary algorithms employed by vehicle OEMs, which generally base such estimations on expected vehicle operational parameters, rather than actual vehicle operational parameters. As well, because odometer data and engine oil quality data from the current oil cycle are used, the vehicle operational factors of another, previous oil cycle, which may differ, may not be considered during the estimation. As a result, the systems and methods of the present disclosure may estimate the remaining useful life of vehicle engine oil based on current vehicle operational factors, independently of any previous vehicle operational factors (i.e., those of a previous oil cycle), which, if different, may have affected the accuracy of the estimation.

In the present disclosure, all terms referred to in singular form are meant to encompass plural forms of the same. Likewise, all terms referred to in plural form are meant to encompass singular forms of the same. Unless defined otherwise, all technical and scientific terms used herein have the same meaning as commonly understood by one of ordinary skill in the art to which this disclosure pertains.

As used herein, the term "about" refers to an approximately +/-10 % variation from a given value. It is to be understood that such a variation is always included in any given value provided herein, whether or not it is specifically referred to.

It should be understood that the compositions and methods are described in terms of "comprising," "containing," or "including" various components or steps, the compositions and methods can also "consist essentially of or "consist of the various components and steps. Moreover, the indefinite articles "a" or "an," as used in the claims, are defined herein to mean one or more than one of the element that it introduces.

Throughout this specification and the appended claims, infinitive verb forms are often used, such as "to operate" or "to couple". Unless context dictates otherwise, such infinitive verb forms are used in an open and inclusive manner, such as "to at least operate" or "to at least couple".

For the sake of brevity, only certain ranges are explicitly disclosed herein. However, ranges from any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited. Additionally, whenever a numerical range with a lower limit and an upper limit is disclosed, any number and any included range falling within the range are specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values even if not explicitly recited. Thus, every point or individual value may serve as its own lower or upper limit combined with any other point or individual value or any other lower or upper limit, to recite a range not explicitly recited.

The Drawings are not necessarily to scale and may be illustrated by phantom lines, diagrammatic representations, and fragmentary views. In certain instances, details that are not necessary for an understanding of the exemplary embodiments or that render other details difficult to perceive may have been omitted.

The specification includes various implementations in the form of block diagrams, schematics, and flowcharts. A person of skill in the art will appreciate that any function or operation within such block diagrams, schematics, and flowcharts can be implemented by a wide range of hardware, software, firmware, or combination thereof. As non-limiting examples, the various embodiments herein can be implemented in one or more of: application-specific integrated circuits (ASICs), standard integrated circuits (ICs), programmable logic devices (PLDs), field-programmable gate arrays (FPGAs), computer programs executed by any number of computers or processors, programs executed by one or more control units or processor units, firmware, or any combination thereof.

The disclosure includes descriptions of several processors. Said processors can be implemented as any hardware capable of processing data, such as application-specific integrated circuits (ASICs), standard integrated circuits (ICs), programmable logic devices (PLDs), field-programmable gate arrays (FPGAs), logic circuits, or any other appropriate hardware. The disclosure also includes descriptions of several non-transitory processor-readable storage mediums. Said non-transitory processor-readable storage mediums can be implemented as any hardware capable of storing data, such as magnetic drives, flash drives, RAM, or any other appropriate data storage hardware. Further, mention of data or information being stored at a device generally refers to the data information being stored at a non-transitory processor-readable storage medium of said device.

Therefore, the present disclosure is well adapted to attain the ends and advantages mentioned as well as those that are inherent therein. The particular embodiments disclosed above are illustrative only, as the present disclosure may be modified and practiced in different but equivalent manners apparent to those skilled in the art having the benefit of the teachings herein. Although individual embodiments are dis-cussed, the disclosure covers all combinations of all those embodiments. Furthermore, no limitations are intended to the details of construction or design herein shown, other than as described in the claims below. Also, the terms in the claims have their plain, ordinary meaning unless otherwise explicitly and clearly defined by the patentee. It is therefore evident that the particular illustrative embodiments disclosed above may be altered or modified and all such variations are considered within the scope and spirit of the present disclosure. If there is any conflict in the usages of a word or term in this specification and one or more patent(s) or other documents that may be incorporated herein by reference, the definitions that are consistent with this specification should be adopted.

Many obvious variations of the embodiments set out herein will suggest themselves to those skilled in the art in light of the present disclosure. Such obvious variations are within the full intended scope of the appended claims.

## Claims

1. A system (110, 130, 150) for estimating a remaining useful life of vehicle engine oil, the system (110, 130, 150) comprising:

    at least one data storage (114, 134) operable to store telematics data originating from a plurality of vehicles (120), the telematics data comprising odometer data and engine oil quality data (510, 520) associated with each of the plurality of vehicles (120); and
    at least one processor (112, 132, 152) in communication with the at least one data storage, the at least one processor (112, 132, 152) operable to:

        receive the telematics data;
        determine, for one or more of the plurality of vehicles (120), a start point of a current oil cycle based on when an oil cycle event previously occurred by identifying within the engine oil quality data (510, 520):

        a first series of engine oil quality datapoints (512, 522), and
        a second series of engine oil quality datapoints (516, 526) immediately following the first series,
        a difference between a final datapoint (514, 524) of the first series (512, 522) and an initial datapoint (518, 528) of the second series (516, 526) being greater than a predetermined threshold;

    synchronize the odometer data and the engine oil quality data (510, 520) by associating odometer datapoints and engine oil quality datapoints (510, 520) reported within a same time period;
    determine a remaining useful vehicle engine oil distance by applying to synchronized odometer and engine oil quality data (510, 520) reported during the current oil cycle a regression model; and
    determine a remaining useful vehicle engine oil time by converting the remaining useful vehicle engine oil distance thereto based on an average difference in odometer datapoints reported during a selected time period,

    whereby the remaining useful life of vehicle engine oil is estimated based on vehicle operational factors associated with the current oil cycle, and independently from those of a previous oil cycle.

2. The system (110, 130, 150) of claim 1, wherein each datapoint of the first series (512, 522) and the second series (516, 526) is greater than an immediately preceding datapoint within a predetermined threshold, less than the immediately preceding datapoint within a predetermined threshold, or equal to the immediately preceding datapoint.

3. The system (110, 130, 150) of claim 1 or 2, wherein the at least one processor (112, 132, 152) is operable to determine the start point of the current oil cycle by:

    selecting engine oil quality data (510, 520) reported within a selected reporting period; and
    identifying within the engine oil quality data (510, 520) reported within the selected reporting period the first series (512, 522) and the second series (516, 526).

**4.** The system (110, 130, 150) of any one of claims 1 to 3, wherein the at least one processor (112, 132, 152) is operable to determine the remaining useful vehicle engine oil distance by:

determining from the regression model at least a rate of degradation of the engine oil quality datapoints;

determining, based on the rate of degradation, an odometer reading at which the engine oil quality datapoints indicate engine oil should be changed; and

determining the remaining useful vehicle engine oil distance based on a difference between a most recent odometer reading and the odometer datapoint at which the engine oil quality datapoints indicate engine oil should be changed.

**5.** The system (110, 130, 150) of claim 4, wherein the at least one processor (112, 132, 152) is further operable to determine confidence bounds for the rate of degradation based on a residual standard deviation thereof and a confidence coefficient.

**6.** The system (110, 130, 150) of claim 5, wherein the at least one processor (112, 132, 152) is operable to: determine the confidence coefficient based on one or more previous oil cycles of the one or more of the plurality of vehicles (120), select the confidence coefficient based on one or more vehicle parameters, or a combination thereof.

**7.** The system (110, 130, 150) of any one of claims 1 to 6, wherein:

the telematics data further comprises vehicle identifying information associated with the plurality of vehicles (120); and
the at least one processor (112, 132, 152) is further operable to determine the remaining useful vehicle engine oil distance by:

identifying, using the vehicle identifying information, a vehicle type of each of the one or more of the plurality of vehicles (120);
selecting, for each of the one or more of the plurality of vehicles (120), a type of regression model based on the vehicle type of each thereof; and
applying the regression model to the synchronized odometer and engine oil quality data (510, 520) reported during the current oil cycle.

**8.** A method for estimating a remaining useful life of vehicle engine oil, the method comprising operating at least one processor (112, 132, 152) to:

receive telematics data originating from a plurality of vehicles (120), the telematics data comprising odometer data and engine oil quality data (510, 520) associated with each of the plurality of vehicles (120);
determine, for one or more of the plurality of vehicles (120), a start point of a current oil cycle based on when an oil cycle event previously occurred by identifying within the engine oil quality data (510, 520):

a first series of engine oil quality datapoints (512, 522), and
a second series of engine oil quality datapoints (516, 526) immediately following the first series (512, 522),
a difference between a final datapoint (514, 524) of the first series (512, 522) and an initial datapoint (518, 528) of the second series (516, 526) being greater than a predetermined threshold;

synchronize the odometer data and the engine oil quality data (510, 520) by associating odometer datapoints and engine oil quality datapoints reported within a same time period;
determine a remaining useful vehicle engine oil distance by applying to synchronized odometer and engine oil quality data (510, 520) reported during the current oil cycle a regression model; and
determine a remaining useful vehicle engine oil time by converting the remaining useful vehicle engine oil distance thereto based on an average difference in odometer datapoints reported during a selected time period,
whereby the remaining useful life of vehicle engine oil is estimated based on vehicle operational factors associated with the current oil cycle, and independently from those of a previous oil cycle.

**9.** The method of claim 8, wherein each datapoint of the first series (512, 522) and the second series (516, 526) is greater than an immediately preceding datapoint within a predetermined threshold, less than the immediately preceding datapoint within a predetermined threshold, or equal to the immediately preceding datapoint.

**10.** The method of claim 8 or 9, wherein the determining of the start point of the current oil cycle comprises operating the at least one processor (112, 132, 152) to:

select engine oil quality data (510, 520) reported within a selected reporting period; and
identify within the engine oil quality data (510, 520) reported within the selected reporting period the first series (512, 522) and the second series (516, 526).

11. The method of any one of claims 8 to 10, wherein the determining of the remaining useful vehicle engine oil distance comprises operating the at least one processor (112, 132, 152) to:

> determine from the regression model at least a rate of degradation of the engine oil quality datapoints;
> determine, based on the rate of degradation, an odometer reading at which the engine oil quality datapoints indicate engine oil should be changed; and
> determine the remaining useful vehicle engine oil distance based on a difference between a most recent odometer datapoint and the odometer reading at which the engine oil quality datapoints indicate engine oil should be changed.

12. The method of claim 11, further comprising operating the at least one processor (112, 132, 152) to determine confidence bounds for the rate of degradation based on a residual standard deviation thereof and a confidence coefficient.

13. The method of claim 12, comprising operating the at least one processor (112, 132, 152) to: determine the confidence coefficient based on one or more previous oil cycles of the one or more of the plurality of vehicles (120), select the confidence coefficient based on one or more vehicle parameters.

14. The method of any one of claims 8 to 13, wherein:

> the telematics data further comprises vehicle identifying information associated with the plurality of vehicles (120); and
> the determining of the remaining useful vehicle engine oil distance further comprises operating the at least one processor (112, 132, 152) to:
>
> > identifying, using the vehicle identifying information, a vehicle type of each of the one or more of the plurality of vehicles (120);
> > selecting, for each of the one or more of the plurality of vehicles (120), a type of regression model based on the vehicle type of each thereof; and
> > applying the regression model to the synchronized odometer and engine oil quality data (510, 520) reported during the current oil cycle.

15. A non-transitory computer readable medium having instructions stored thereon executable by at least one processor (112, 132, 152) to implement a method for estimating a remaining useful life of vehicle engine oil according to any of claims 8 to 14.

**Patentansprüche**

1. System (110, 130, 150) zur Schätzung einer verbleibenden Lebensdauer von Fahrzeugmotoröl, wobei das System (110, 130, 150) Folgendes umfasst:

> zumindest einen Datenspeicher (114, 134), der betrieben werden kann, um Telematikdaten stammend aus einer Vielzahl von Fahrzeugen (120) zu speichern, wobei die Telematikdaten Kilometerzählerdaten und Daten der Motorölqualität (510, 520) umfassen, die mit jedem der Vielzahl von Fahrzeugen (120) assoziiert sind; und
> zumindest einen Prozessor (112, 132, 152) in Kommunikation mit dem zumindest einen Datenspeicher, wobei der zumindest eine Prozessor (112, 132, 152) betrieben werden kann, um:
>
> > die Telematikdaten zu empfangen;
> > für ein oder mehrere der Vielzahl von Fahrzeugen (120), einen Startpunkt eines aktuellen Ölkreislaufs zu bestimmen, basierend darauf, wann ein Ölkreislaufereignis zuvor stattfand, indem innerhalb der Daten der Motorölqualität (510, 520) Folgendes identifiziert wird:
> >
> > > eine erste Reihe von Datenpunkten der Motorölqualität (512, 522), und
> > > eine zweite Reihe von Datenpunkten der Motorölqualität (516, 526), die der ersten Reihe unmittelbar folgen,
> > > ein Unterschied zwischen einem letzten Datenpunkt (514, 524) der ersten Reihe (512, 522) und einem anfänglichen Datenpunkt (518, 528) der zweiten Reihe (516, 526), der größer als eine vorbestimmte Schwelle ist;
> >
> > die Kilometerzählerdaten und die Daten der Motorölqualität (510, 520) zu synchronisieren, indem Kilometerzählerdatenpunkte und Datenpunkte der Motorölqualität (510, 520), die innerhalb eines gleichen Zeitraums gemeldet werden, assoziiert werden;
> > eine verbleibende nützliche Strecke des Fahrzeugmotoröls zu bestimmen, indem auf die synchronisierten Kilometerzählerdaten und Daten der Motorölqualität (510, 520), die während des aktuellen Ölkreislaufs gemeldet werden, ein Regressionsmodell angewendet wird; und

eine verbleibende nützliche Zeit des Fahrzeugmotoröls zu bestimmen, indem die verbleibende nützliche Strecke des Fahrzeugmotoröls darin umgewandelt wird, basierend auf einem durchschnittlichen Unterschied in den Kilometerzählerdatenpunkten, die während eines ausgewählten Zeitraums gemeldet werden,

wodurch die verbleibende Lebensdauer von Fahrzeugmotoröl basierend auf Betriebsfaktoren des Fahrzeugs, die mit dem aktuellen Ölkreislauf assoziiert sind, und unabhängig von denjenigen eines vorherigen Ölkreislaufs, geschätzt wird.

2. System (110, 130, 150) nach Anspruch 1, wobei jeder Datenpunkt der ersten Reihe (512, 522) und der zweiten Reihe (516, 526) größer als ein unmittelbar vorheriger Datenpunkt innerhalb einer vorbestimmten Schwelle, kleiner als der unmittelbar vorherige Datenpunkt innerhalb einer vorbestimmten Schwelle oder dem unmittelbar vorherigen Datenpunkt gleich ist.

3. System (110, 130, 150) nach Anspruch 1 oder 2, wobei der zumindest eine Prozessor (112, 132, 152) betrieben werden kann, um den Startpunkt des aktuellen Ölkreislaufs zu bestimmen, indem:

Daten der Motorölqualität (510, 520), die innerhalb eines ausgewählten Meldezeitraums gemeldet werden, ausgewählt werden; und innerhalb der Daten der Motorölqualität (510, 520), die innerhalb des ausgewählten Meldezeitraums gemeldet werden, die erste Reihe (512, 522) und die zweite Reihe (516, 526) identifiziert werden.

4. System (110, 130, 150) nach einem der Ansprüche 1 bis 3, wobei der zumindest eine Prozessor (112, 132, 152) betrieben werden kann, um die verbleibende nützliche Strecke des Fahrzeugmotoröls zu bestimmen, indem:

aus dem Regressionsmodell zumindest eine Abbaurate der Datenpunkte der Motorölqualität bestimmt wird;
basierend auf der Abbaurate, eine Kilometerzählerablesung bestimmt wird, bei welcher die Datenpunkte der Motorölqualität darauf hindeuten, dass das Motoröl gewechselt werden soll; und
die verbleibende nützliche Strecke des Fahrzeugmotoröls bestimmt wird, basierend auf einem Unterschied zwischen einer aktuellsten Kilometerzählerablesung und dem Kilometerzählerdatenpunkt, bei welchem die Datenpunkte der Motorölqualität darauf hindeuten, dass das Motoröl gewechselt werden soll.

5. System (110, 130, 150) nach Anspruch 4, wobei der zumindest eine Prozessor (112, 132, 152) zusätzlich betrieben werden kann, um Konfidenzgrenzen für die Abbaurate basierend auf einer Reststandardabweichung derselben und einem Konfidenzkoeffizienten zu bestimmen.

6. System (110, 130, 150) nach Anspruch 5, wobei der zumindest eine Prozessor (112, 132, 152) betrieben werden kann, um: den Konfidenzkoeffizienten basierend auf einem oder mehreren vorherigen Ölkreisläufen des einen oder der mehreren der Vielzahl von Fahrzeugen (120) zu bestimmen, den Konfidenzkoeffizienten basierend auf einem oder mehreren Fahrzeugparametern auszuwählen oder eine Kombination derselben.

7. System (110, 130, 150) nach einem der Ansprüche 1 bis 6, wobei:

die Telematikdaten zusätzlich Identifizierungsinformation von Fahrzeugen umfassen, die mit der Vielzahl von Fahrzeugen (120) assoziiert ist; und
der zumindest eine Prozessor (112, 132, 152) zusätzlich betrieben werden kann, um die verbleibende nützliche Strecke des Fahrzeugmotoröls zu bestimmen, indem:

unter Verwendung der Identifizierungsinformation von Fahrzeugen, ein Fahrzeugtyp von jedem des einen oder der mehreren der Vielzahl von Fahrzeugen (120) identifiziert wird;
für jedes des einen oder der mehreren der Vielzahl von Fahrzeugen (120), ein Typ von Regressionsmodell basierend auf dem Fahrzeugtyp von jedem derselben ausgewählt wird; und
das Regressionsmodell auf die synchronisierten Kilometerzählerdaten und Daten der Motorölqualität (510, 520), die während des aktuellen Ölkreislaufs gemeldet werden, angewendet wird.

8. Verfahren zur Schätzung einer verbleibenden Lebensdauer von Fahrzeugmotoröl, wobei das Verfahren das Betreiben zumindest eines Prozessors (112, 132, 152) umfasst, um:

Telematikdaten stammend aus einer Vielzahl von Fahrzeugen (120) zu empfangen, wobei die Telematikdaten Kilometerzählerdaten und Daten der Motorölqualität (510, 520) umfassen, die mit jedem der Vielzahl von Fahrzeugen (120) assoziiert sind;

für ein oder mehrere der Vielzahl von Fahrzeugen (120), einen Startpunkt eines aktuellen Ölkreislaufs zu bestimmen, basierend darauf, wann ein Ölkreislaufereignis zuvor stattfand, indem innerhalb der Daten der Motorölqualität (510, 520) Folgendes identifiziert wird:

eine erste Reihe von Datenpunkten der Motorölqualität (512, 522), und eine zweite Reihe von Datenpunkten der Motorölqualität (516, 526), die der ersten Reihe (512, 522) unmittelbar folgen, einen Unterschied zwischen einem letzten Datenpunkt (514, 524) der ersten Reihe (512, 522) und einem anfänglichen Datenpunkt (518, 528) der zweiten Reihe (516, 526), der größer als eine vorbestimmte Schwelle ist;

die Kilometerzählerdaten und die Daten der Motorölqualität (510, 520) zu synchronisieren, indem Kilometerzählerdatenpunkte und Datenpunkte der Motorölqualität, die innerhalb eines gleichen Zeitraums gemeldet werden, assoziiert werden;

eine verbleibende nützliche Strecke des Fahrzeugmotoröls zu bestimmen, indem auf die synchronisierten Kilometerzählerdaten und Daten der Motorölqualität (510, 520), die während des aktuellen Ölkreislaufs gemeldet werden, ein Regressionsmodell angewendet wird; und

eine verbleibende nützliche Zeit des Fahrzeugmotoröls zu bestimmen, indem die verbleibende nützliche Strecke des Fahrzeugmotoröls darin umgewandelt wird, basierend auf einem durchschnittlichen Unterschied bei den Kilometerzählerdatenpunkten, die während eines ausgewählten Zeitraums gemeldet werden,

wodurch die verbleibende Lebensdauer von Fahrzeugmotoröl basierend auf Betriebsfaktoren des Fahrzeugs, die mit dem aktuellen Ölkreislauf assoziiert sind, und unabhängig von denjenigen eines vorherigen Ölkreislaufs, geschätzt wird.

9. Verfahren nach Anspruch 8, wobei jeder Datenpunkt der ersten Reihe (512, 522) und der zweiten Reihe (516, 526) größer als ein unmittelbar vorheriger Datenpunkt innerhalb einer vorbestimmten Schwelle, kleiner als der unmittelbar vorherige Datenpunkt innerhalb einer vorbestimmten Schwelle oder dem unmittelbar vorherigen Datenpunkt gleich ist.

10. Verfahren nach Anspruch 8 oder 9, wobei das Bestimmen des Startpunktes des aktuellen Ölkreislaufs das Betreiben des zumindest eines Prozessors (112, 132, 152) umfasst, um:

Daten der Motorölqualität (510, 520), die innerhalb eines ausgewählten Meldezeitraums gemeldet werden, auszuwählen; und innerhalb der Daten der Motorölqualität (510, 520), die innerhalb des ausgewählten Meldezeitraums gemeldet werden, die erste Reihe (512, 522) und die zweite Reihe (516, 526) zu identifizieren.

11. Verfahren nach einem der Ansprüche 8 bis 10, wobei das Bestimmen der verbleibenden nützlichen Strecke des Fahrzeugmotoröls das Betreiben des zumindest einen Prozessors (112, 132, 152) umfasst, um:

aus dem Regressionsmodell zumindest eine Abbaurate der Datenpunkte der Motorölqualität zu bestimmen; basierend auf der Abbaurate, eine Kilometerzählerablesung zu bestimmen, bei welcher die Datenpunkte der Motorölqualität darauf hindeuten, dass das Motoröl gewechselt werden soll; und die verbleibende nützliche Strecke des Fahrzeugmotoröls zu bestimmen, basierend auf einem Unterschied zwischen einem aktuellsten Kilometerzählerdatenpunkt und der Kilometerzählerablesung, bei welcher die Datenpunkte der Motorölqualität darauf hindeuten, dass das Motoröl gewechselt werden soll.

12. Verfahren nach Anspruch 11, das zusätzlich das Betreiben des zumindest einen Prozessors (112, 132, 152) umfasst, um Konfidenzgrenzen für die Abbaurate basierend auf einer Reststandardabweichung derselben und einem Konfidenzkoeffizienten zu bestimmen.

13. Verfahren nach Anspruch 12, das das Betreiben des zumindest einen Prozessors (112, 132, 152) umfasst, um: den Konfidenzkoeffizienten basierend auf einem oder mehreren vorherigen Ölkreisläufen des einen oder der mehreren der Vielzahl von Fahrzeugen (120) zu bestimmen oder den Konfidenzkoeffizienten basierend auf einem oder mehreren Fahrzeugparametern auszuwählen.

14. Verfahren nach einem der Ansprüche 8 bis 13, wobei:

die Telematikdaten zusätzlich Identifizierungsinformation von Fahrzeugen umfassen, die mit der Vielzahl von Fahrzeugen (120) assoziiert sind; und das Bestimmen der verbleibenden nützlichen Strecke des Fahrzeugmotoröls zusätzlich das Betreiben des zumindest einen Prozessors (112, 132, 152) umfasst, um:

unter Verwendung der Identifizierungsinformation von Fahrzeugen, ein Fahrzeugtyp von jedem des einen oder der mehreren der Vielzahl von Fahrzeugen (120) zu identifizieren;

für jedes des einen oder der mehreren der Vielzahl von Fahrzeugen (120), ein Typ von Regressionsmodell basierend auf dem Fahrzeugtyp von jedem derselben auszuwählen; und

das Regressionsmodell auf die synchronisierten Kilometerzählerdaten und Daten der Motorölqualität (510, 520), die während des aktuellen Ölkreislaufs gemeldet werden, anzuwenden.

15. Nichtflüchtiges computerlesbares Medium, das darin gespeicherte Anweisungen aufweist, die vom zumindest einen Prozessor (112, 132, 152) ausgeführt werden können, um ein Verfahren zur Schätzung einer verbleibenden Lebensdauer von Fahrzeugmotoröl nach einem der Ansprüche 8 bis 14 zu implementieren.

## Revendications

1. Système (110, 130, 150) d'estimation d'une durée de vie utile restante d'huile de moteur de véhicule, le système (110, 130, 150) comprenant :

au moins un stockage de données (114, 134) pouvant être utilisé pour stocker des données télématiques provenant d'une pluralité de véhicules (120), les données télématiques comprenant des données d'odomètre et des données de qualité de l'huile de moteur (510, 520) associées à chacun de la pluralité de véhicules (120) ; et

au moins un processeur (112, 132, 152) en communication avec l'au moins un stockage de données, l'au moins un processeur (112, 132, 152) pouvant être utilisé pour :

recevoir les données télématiques ;

déterminer, pour un ou plusieurs de la pluralité de véhicules (120), un point de départ d'un cycle d'huile actuel en fonction du moment où un événement de cycle d'huile s'est produit précédemment, en identifiant dans les données de qualité de l'huile de moteur (510, 520) :

une première série de points de données de qualité de l'huile de moteur (512, 522) et

une deuxième série de points de données de qualité de l'huile de moteur (516, 526) suivant immédiatement la première série,

une différence entre un point de données final (514, 524) de la première série (512, 522) et un point de données initial (518, 528) de la deuxième série (516, 526) étant supérieure à un seuil prédéterminé ;

synchroniser les données d'odomètre et les données de qualité de l'huile de moteur (510, 520) en associant les points de données d'odomètre et les points de données de qualité de l'huile de moteur (510, 520) notifiées au cours d'une même période de temps ;

déterminer une distance d'huile de moteur de véhicule utile restante en appliquant aux données d'odomètre et de qualité de l'huile de moteur synchronisées (510, 520) notifiés lors du cycle d'huile actuel un modèle de régression ; et

déterminer un temps d'huile de moteur de véhicule utile restant en convertissant la distance d'huile de moteur de véhicule utile restante en celui-ci en fonction d'une différence moyenne de points de données d'odomètre notifiés au cours d'une période de temps sélectionnée,

par lequel la durée de vie utile restante d'huile de moteur de véhicule est estimée en fonction de facteurs de fonctionnement de véhicule associés au cycle d'huile actuel et indépendamment de ceux d'un cycle d'huile précédent.

2. Système (110, 130, 150) selon la revendication 1, dans lequel chaque point de données de la première série (512, 522) et de la deuxième série (516, 526) est supérieur à un point de données immédiatement précédent dans un seuil prédéterminé, inférieur au point de données immédiatement précédent dans un seuil prédéterminé ou égal au point de données immédiatement précédent.

3. Système (110, 130, 150) selon revendication 1 ou 2, dans lequel l'au moins un processeur (112, 132, 152) peut être utilisé pour déterminer le point de départ du cycle d'huile actuel par :

sélectionner des données de qualité de l'huile de moteur (510, 520) notifiés au cours d'une période de notification sélectionnée ; et

identifier dans les données de qualité de l'huile de moteur (510, 520) notifiés au cours de la période de notification sélectionnée la première série (512, 522) et la deuxième série (516, 526).

4. Système (110, 130, 150) selon l'une quelconque des revendications 1 à 3, dans lequel l'au moins un processeur (112, 132, 152) peut être utilisé pour déterminer la distance d'huile de moteur de véhicule utile restante par :

déterminer, à partir du modèle de régression, au moins un taux de dégradation des points de données de qualité de l'huile de moteur ; déterminer, en fonction du taux de dégradation, une lecture d'odomètre à laquelle les points de données de qualité de l'huile de moteur indiquent que l'huile de moteur doit être changée ; et déterminer la distance d'huile de moteur de véhicule utile restante en fonction d'une différence entre une lecture d'odomètre plus récente et le point de données d'odomètre auquel les points de données de qualité de l'huile de moteur indiquent que l'huile de moteur doit être changée.

5. Système (110, 130, 150) selon la revendication 4, dans lequel l'au moins un processeur (112, 132, 152) peut être utilisé en outre pour déterminer des limites de confiance pour le taux de dégradation en fonction d'un écart type résiduel de celui-ci et d'un coefficient de confiance.

6. Système (110, 130, 150) selon la revendication 5, dans lequel l'au moins un processeur (112, 132, 152) peut être utilisé pour : déterminer le coefficient de confiance en fonction d'un ou plusieurs cycles d'huile précédents de l'un ou plusieurs de la pluralité de véhicules (120), sélectionner le coefficient de confiance en fonction d'un ou plusieurs paramètres de véhicule ou une combinaison de ceux-ci.

7. Système (110, 130, 150) selon l'une quelconque des revendications 1 à 6, dans lequel :

les données télématiques comprennent en outre des informations d'identification de véhicule associées avec la pluralité de véhicules (120) ; et l'au moins un processeur (112, 132, 152) peut être utilisé en outre pour déterminer la distance d'huile de moteur de véhicule utile restante par :

identifier, en utilisant les informations d'identification de véhicule, un type de véhicule de chacun de l'un ou plusieurs de la pluralité de véhicules (120) ; sélectionner, pour chacun de l'un ou plusieurs de la pluralité de véhicules (120), un type de modèle de régression en fonction du type de véhicule de chacun de ceux-ci ; et appliquer le modèle de régression aux données d'odomètre et de qualité de l'huile de moteur synchronisées (510, 520) notifiés lors du cycle d'huile actuel.

8. Procédé d'estimation d'une durée de vie utile restante d'huile de moteur de véhicule, le procédé comprenant l'utilisation d'au moins un processeur (112, 132, 152) pour :

recevoir des données télématiques provenant d'une pluralité de véhicules (120), les données télématiques comprenant des données d'odomètre et des données de qualité de l'huile de moteur (510, 520) associées à chacun de la pluralité de véhicules (120) ; déterminer, pour un ou plusieurs de la pluralité de véhicules (120), un point de départ d'un cycle d'huile actuel en fonction du moment où un événement de cycle d'huile s'est produit précédemment, en identifiant dans les données de qualité de l'huile de moteur (510, 520) :

une première série de points de données de qualité de l'huile de moteur (512, 522) et une deuxième série de points de données de qualité de l'huile de moteur (516, 526) suivant immédiatement la première série (512, 522), une différence entre un point de données final (514, 524) de la première série (512, 522) et un point de données initial (518, 528) de la deuxième série (516, 526) étant supérieure à un seuil prédéterminé ;

synchroniser les données d'odomètre et les données de qualité de l'huile de moteur (510, 520) en associant les points de données d'odomètre et les points de données de qualité de l'huile de moteur notifiées au cours d'une même période de temps ; déterminer une distance d'huile de moteur de véhicule utile restante en appliquant aux données d'odomètre et de qualité de l'huile de moteur synchronisées (510, 520) notifiés lors du cycle d'huile actuel un modèle de régression ; et déterminer un temps d'huile de moteur de véhicule utile restant en convertissant la distance d'huile de moteur de véhicule utile restante en celui-ci en fonction d'une différence moyenne de points de données d'odomètre notifiés au cours d'une période de temps sélectionnée, par lequel la durée de vie utile restante d'huile de moteur de véhicule est estimée en fonction de facteurs de fonctionnement de véhicule associés au cycle d'huile actuel et indépendamment de ceux d'un cycle d'huile précédent.

9. Procédé selon la revendication 8, dans lequel chaque point de données de la première série (512, 522) et de la deuxième série (516, 526) est supérieur à un point de données immédiatement précédent dans un seuil prédéterminé, inférieur au point de données immédiatement précédent dans un seuil prédéterminé ou égal au point de données immédiatement précédent.

10. Procédé selon la revendication 8 ou 9, dans lequel la détermination du point de départ du cycle d'huile actuel comprend l'utilisation de l'au moins un processeur (112, 132, 152) pour :

sélectionner des données de qualité de l'huile de moteur (510, 520) notifiés au cours d'une période de notification sélectionnée ; et identifier dans les données de qualité de l'huile de moteur (510, 520) notifiés au cours de la période de notification sélectionnée la première série (512, 522) et la deuxième série (516, 526).

11. Procédé selon l'une quelconque des revendications 8 à 10, dans lequel la détermination de la distance d'huile de moteur de véhicule utile restante comprend l'utilisation de l'au moins un processeur (112, 132, 152) pour :

déterminer, à partir du modèle de régression, au moins un taux de dégradation des points de données de qualité de l'huile de moteur ; déterminer, en fonction du taux de dégradation, une lecture d'odomètre à laquelle les points de données de qualité de l'huile de moteur indiquent que l'huile de moteur doit être changée ; et déterminer la distance d'huile de moteur de véhicule utile restante en fonction d'une différence entre un point de données d'odomètre plus récent et la lecture d'odomètre à laquelle les points de données de qualité de l'huile de moteur indiquent que l'huile de moteur doit être changée.

12. Procédé selon la revendication 11, comprenant en outre l'utilisation de l'au moins un processeur (112, 132, 152) pour déterminer des limites de confiance pour le taux de dégradation en fonction d'un écart type résiduel de celui-ci et d'un coefficient de confiance.

13. Procédé selon la revendication 12, comprenant l'utilisation de l'au moins un processeur (112, 132, 152) pour : déterminer le coefficient de confiance en fonction d'un ou plusieurs cycles d'huile précédents de l'un ou plusieurs de la pluralité de véhicules (120) et sélectionner le coefficient de confiance en fonction d'un ou plusieurs paramètres de véhicule.

14. Procédé selon l'une quelconque des revendications 8 à 13, dans lequel :

les données télématiques comprennent en outre des informations d'identification de véhicule associées avec la pluralité de véhicules (120) ; et la détermination de la distance d'huile de moteur de véhicule utile restante comprend en outre l'utilisation de l'au moins un processeur (112, 132, 152) pour :

identifier, en utilisant les informations d'identification de véhicule, un type de véhicule de chacun de l'un ou plusieurs de la pluralité de véhicules (120) ; sélectionner, pour chacun de l'un ou plusieurs de la pluralité de véhicules (120), un type de modèle de régression en fonction du type de véhicule de chacun de ceux-ci ; et appliquer le modèle de régression aux données d'odomètre et de qualité de l'huile de moteur synchronisées (510, 520) notifiés lors du cycle d'huile actuel.

15. Support lisible par ordinateur non transitoire ayant des instructions stockées dans celui-ci exécutables par au moins un processeur (112, 132, 152) pour mettre en œuvre un procédé d'estimation d'une durée de vie utile restante d'huile de moteur de véhicule selon l'une quelconque des revendications 8 à 14.

FIG. 1

**FIG. 2**

110

Fleet Management System

Processor 112

Data Storage 114

Communication Interface 116

140

150

Computing Device

Processor 152

Data Storage 154

Communication Interface 156

Display 158

**FIG. 3**

400

410

RECEIVE TELEMATICS DATA COMPRISING ODOMETER
AND ENGINE OIL QUALITY DATA

420

DETERMINE START POINT OF CURRENT OIL CYCLE
BASED ON WHEN OIL CYCLE EVENT PREVIOUSLY
OCCURRED

430

SYNCHRONIZE ODOMETER AND ENGINE OIL QUALITY
DATA

440

DETERMINE REMAINING USEFUL ENGINE OIL DISTANCE

450

DETERMINE REMAINING USEFUL ENGINE OIL TIME

FIG. 4

FIG. 5A

FIG. 5B

FIG. 5C

FIG. 5

FIG. 6A

FIG. 6B

FIG. 6C

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022412912 A1